(19) Europäisches Patentamt
European Patent Office
Office européen des brevets

(11) Publication number: **0 256 636 B1**

(12) # EUROPEAN PATENT SPECIFICATION

(45) Date of publication of patent specification: **04.03.92**

(51) Int. Cl.$^5$: **C07C 25/18**, C07C 43/18, C07D 239/24, C07D 319/06, C09K 19/30, C09K 19/34

(21) Application number: **87305350.8**

(22) Date of filing: **17.06.87**

The file contains technical information submitted after the application was filed and not included in this specification

(54) **Biphenylyl ethanes and their use in liquid crystal materials and devices.**

(30) Priority: **17.06.86 GB 8614676**

(43) Date of publication of application:
**24.02.88 Bulletin 88/08**

(45) Publication of the grant of the patent:
**04.03.92 Bulletin 92/10**

(84) Designated Contracting States:
**AT BE CH DE FR GB IT LI NL SE**

(56) References cited:
**EP-A- 0 205 998**
**DE-A- 3 233 641**

**CHEMICAL ABSTRACTS, vol. 104, no. 18, 5th May 1986, page 722, abstract no. 159751h, Columbus, Ohio, US; & JP-A-60 163 865**

(73) Proprietor: **The Secretary of State for Defence in Her Britannic Majesty's Government of the United Kingdom of Great Britain and Northern Ireland Whitehall
London SW1A 2HB(GB)**

(72) Inventor: **Gray, George William
"Glenwood" Newsgate Street
Cottingham North Humberside HU16 4D7(GB)**
Inventor: **Toyne, Kenneth Johnson
25 Hall Road
Hull North Humberside HU6 8OW(GB)**
Inventor: **Lacey, David
19 Kildale Close
Hull North Humberside HU8 9NW(GB)**
Inventor: **Poetsch, Eike
Am Buchwald 4
W-6109 Muehltal 6(DE)**
Inventor: **Weber, Georg
Wilhelm Leuschner Strasse
W-6106 Erzhausen(DE)**

(74) Representative: **Lockwood, Peter Brian et al
Procurement Executive Ministry of Defence
Patents 1 A (4) Room 2014, Empress State Building Lillie Road
London SW6 1TR(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

This invention relates to biphenylyl ethanes and their use in liquid crystal materials and devices.

The use of liquid crystal materials to exhibit electro-optical effects in display devices such as digital calculators, watches, meters and simple word displays is now well know. However, known liquid crystal materials are not ideal in all respects and a considerable amount of work is currently being carried out in the art to improve their properties.

Liquid crystal materials normally consist of specially selected mixture compositions and improved materials are obtained by forming new mixtures having an improved combination of properties, e.g. by the provision of new compounds for incorporation in the mixtures. Preferably, such compounds are nematogenic or chiral nematogenic in nature.

The present invention, in particular, relates to fluorinated biphenylyl ethanes.

Some fluorinated analogues of these compounds have been investigated, for example, fluorinated compounds such as

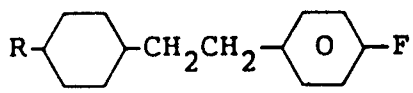

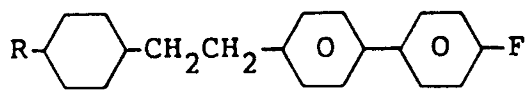

as described in DE A 3233641, and

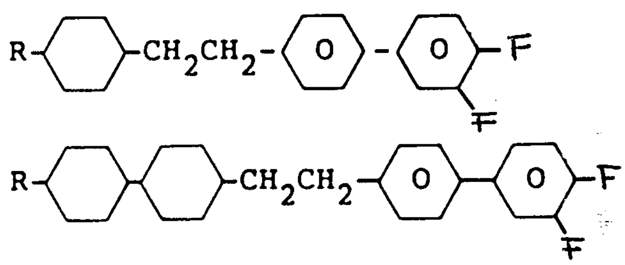

as described in EP A 205998, and

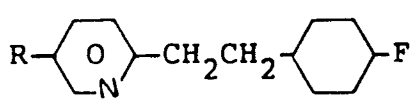

as described in Chemical Abstracts, Vol. 104, 159751h (1986).

To date, the extent to which fluorinated compounds for use as liquid crystal compounds could be investigated has been hampered by the unavailability of suitable starting materials, and the difficulties of organofluorine chemistry.

The present inventors have investigated the field of fluorinated biphenylyl ethanes and have identified a range of novel compounds which are useful components of liquid crystal materials. The compounds are considered novel due to the presence of lateral fluorine substituents which project sideways from the molecule causing a substantial difference to the molecular shape and transverse dipole of the essentially cylindrical non-laterally substituted molecules described above.

According to the present invention there is provided a fluorobiphenylyl ethane suitable for use in liquid crystal materials which has a formula :

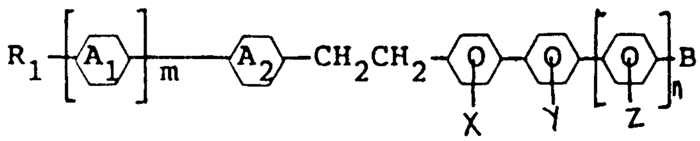

I

wherein :
m represents 0 or 1;
n represents 0 or 1;
$R_1$ represents hydrogen or an alkyl, alkoxy, alkoxy substituted alkyl, alkanoyl, alkanoyloxy, alkoxycarbonyl or alkoxycarbonyloxy group;

each of

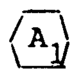 and 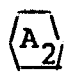

represents a group selected from 1,4-disubstituted cyclohexane with the substituents in the trans configuration; 1,4-disubstituted bicyclo (2,2,2) octane; 2,5-disubstituted 1,3-dioxan with the substituents in the trans configuration; 1,4-disubstituted benzene optionally substituted with fluorine, e.g. in the 2 and/or 3 positions, 2,5-disubstituted 1,3-pyrimidine and 3,6-disubstituted 1,2-pyridazine;

each of X, Y and Z represent hydrogen or fluorine in the 2,3,5 and 6 benzene ring positions provided that at least one of X, Y and Z represents fluorine in at least one ring position; and

B represents a terminal group selected from: fluoro, chloro and $CF_3$;

with the proviso that if rings

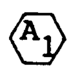 and 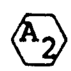

are cyclohexane, and X is hydrogen and Y is fluorine in the 3-position and B is fluorine and $R_1$ is alkyl, then $R_1$ is selected from $C_{4-12}$ chiral alkyl or if m and n are both O then $R_1$ is selected from n-butyl, n-pentyl, n-hexyl and n-octyl, or if m is 1 and n is 0 then $R_1$ is selected from n-propyl, n-butyl, n-pentyl, n-hexyl and n-octyl.

B is preferably fluoro.

$R_1$ is preferably alkyl or alkoxy.

Examples of certain sub-classes of compounds falling within formula I are listed in table 1. Examples of preferred sub-classes of compounds falling within formula I are listed in table 1a i.e. compounds having a general formula Ia;

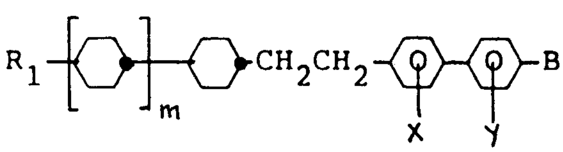

Ia

wherein $R_1$, m and B are as defined in formula I, and where at least one of X or Y is fluorine. In formula Ia, $R_1$ is preferably $C_{1-12}$ alkyl or alkoxy and B is preferably fluorine, and one of X or Y is fluorine, and provided that if Y is fluorine in the 3-position and $R_1$ is alkyl then either $R_1$ is $C_{4-12}$ chiral alkyl, or if m is 0 then $R_1$ is selected from n-butyl, n-pentyl, n-hexyl and n-octyl, or if m is 1 then $R_1$ is selected from n-propyl, n-butyl, n-pentyl, n-hexyl and n-octyl.

Table 1

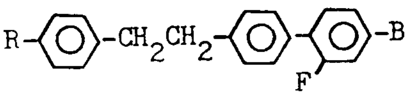 1.1

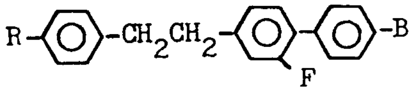 1.2

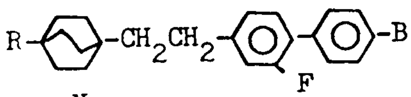 1.3

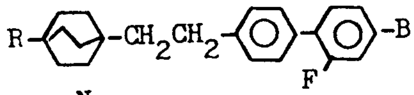 1.4

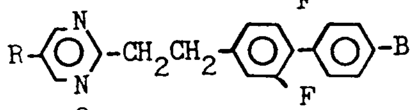 1.5

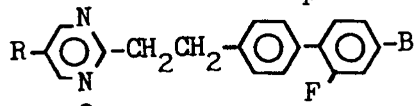 1.6

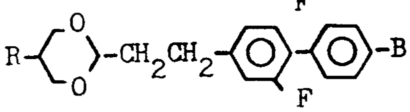 1.7

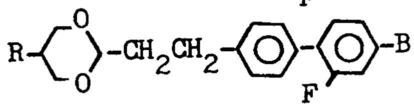 1.8

Table 1a

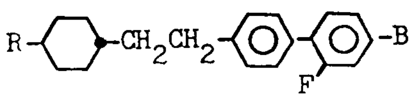 1a1

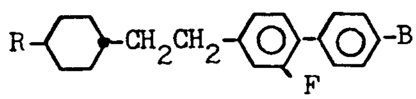 1a4

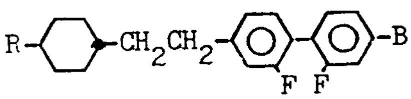 1a2

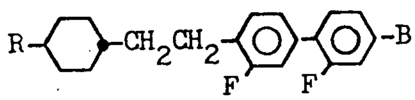 1a5

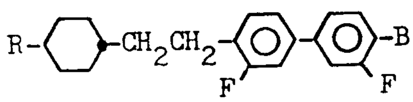 1a3

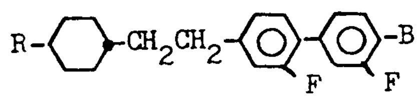 1a6

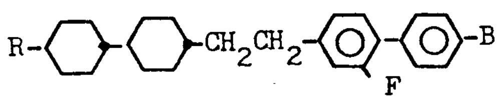 1a7

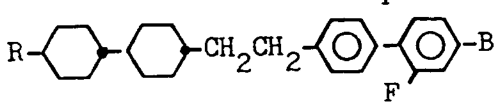 1a8

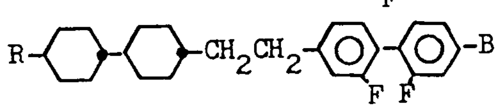 1a9

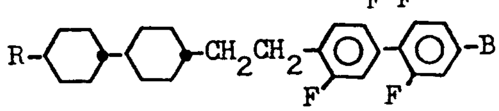 1a10

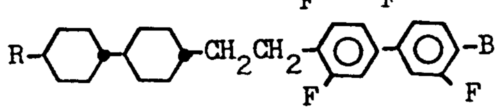 1a11

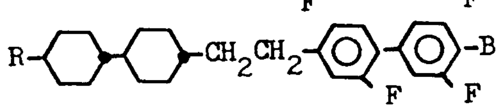 1a12

where R = alkyl or alkoxy.

In the compounds of Formula I where an alkyl group is contained within the group $R_1$ it preferably has from 1 to 12 carbon atoms, and it is preferably an n-alkyl group (where it contains more than 2 carbon atoms). An alkyl group contained within the group $R_1$ may alternatively be a chiral alkyl group having from 4 to 12 carbon atoms, eg (+)-2-methylbutyl.

Preferably, the group $R_1$ has between 1 and 8 carbon atoms.

Generally speaking, the compounds of Formula I are nematogenic or chiral nematogenic (where $R_1$ is chiral) showing a high clearing point (liquid crystal to isotropic liquid transition temperature) and, where n = 0 and m = 0 a relatively low melting point (> 100 ° C), a good chemical and photochemical stability and a positive dielectric anisotropy. They may also advantageously show a reduction in the tendency to form smectic phases by themselves and in admixture with nematogenic compounds of smaller dielectric anisotropy compared with the tendency to form such phases shown by unfluorinated compounds of comparable structure.

They may also show a high clearing point combined with a relatively low viscosity, thus enabling them to be used to raise the clearing point of a liquid crystal mixture in which they are contained.

The compounds of Formula I are therefore useful compounds of liquid crystal compositions for electro-optical displays.

As the compounds of Formula I have a positive dielectric anisotropy they may be added to other liquid crystal materials of positive dielectric anisotropy, or to materials having a lower dielectric anisotropy in order to produce a mixture having amongst other things a suitable dielectric anisotropy. As is well known to those skilled in the art the dielectric anisotropy of the liquid crystal material is necessary to give electro-optical operation and its sign (for a given frequency) is chosen according to the kind of electro-optical device in which the material is to be used.

Compounds of reasonably low melting point are preferred as high dielectric anisotropy components. For example, the low melting compounds of the known classes listed in table 2 are suitable as positive materials for admixture with the compounds of Formula I. In table 2 each R is independently n-alkyl or n-alkoxy and each $R_A$ is independently n-alkyl.

As noted above, the compounds of Formula I may have added to them small dielectric anisotropy compounds, eg to reduce mixture melting point, viscosity or to improve multiplexibility. The classes listed in table 3 are examples of such small dielectric anisotropy compounds.

In table 3 each R is independently n-alkyl or n-alkoxy;

each $R_A$ is independently n-alkyl;

each R′ is independently n-alkyl, n-alkoxy or hydrogen;

x = H or F;

and Q = halogen, eg Cl or F.

In general, one or more compounds of Formula I is likely to be added to one or more compounds of Formula IIIa to IIIi optionally together with one or more compounds of Formula IIa to IIi to form suitable liquid crystal mixtures.

Additional high clearing point compounds may be included in such mixtures eg one or more compounds selected from the classes shown in table 4.

Table 2

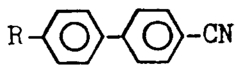
IIa

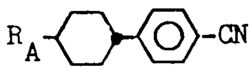
IIb

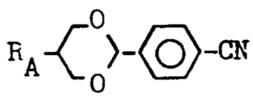
IIc

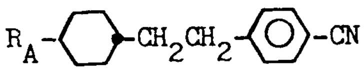
IId

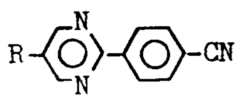
IIe

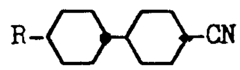
IIf

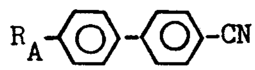
IIg

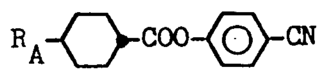
IIh

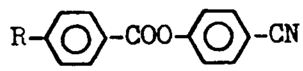
IIi

Table 3

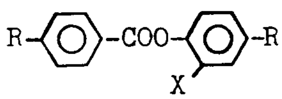
IIIa

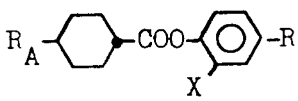
IIIb

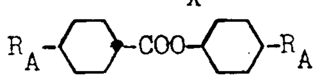
IIIc

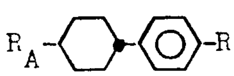
IIId

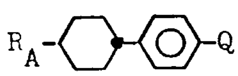
IIIe

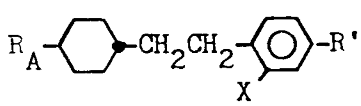
IIIf

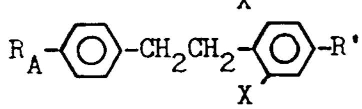
IIIg

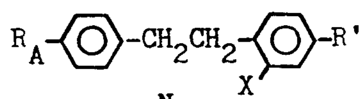
IIIh

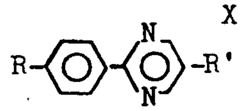
IIIi

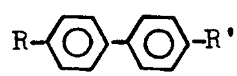
IIIj

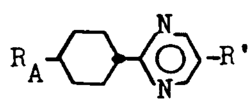
IIIk

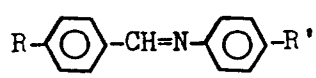
IIIl

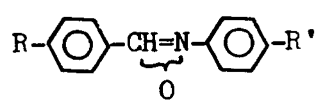
IIIm

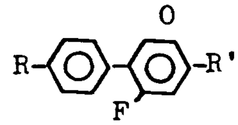
IIIn

Table 4

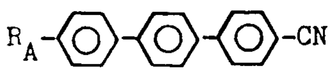 IVa

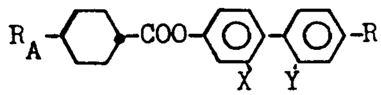 IVh

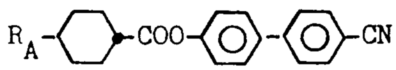 IVb

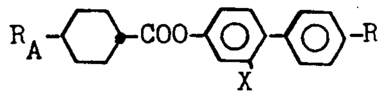 IVi

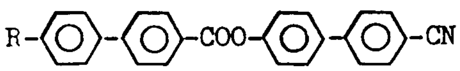 IVc

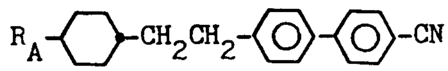 IVj

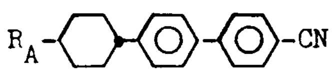 IVd

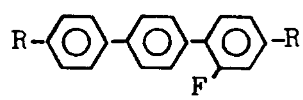 IVk

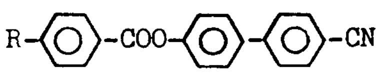 IVe

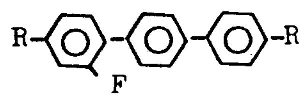 IVl

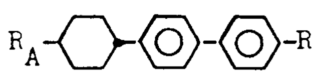 IVf

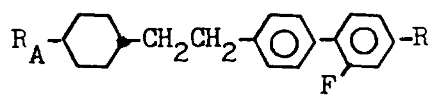 lVm

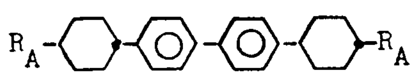 IVg

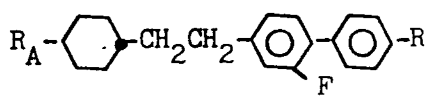 IVn

In table 4 R, $R_A$, and X are as specified above (each one being independently selected).

Other specific known additives, eg low melting chiral additives, such as

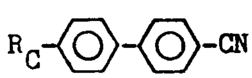 and 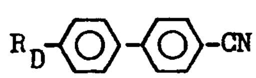

where $R_C$ = (+)-methylbutyl and $R_D$ = (+)-2-methylbutoxy, may be incorporated in the mixture where required.

One or more of compounds according to Formula I may be used in any of the following applications (where material having a positive dielectric anisotropy is referred to as 'positive' material:

(i) as one or more components of a positive nematic material for use in twisted nematic effect devices including multiplexed devices; an example of such a device is given below;

(ii) as one or more components of a positive nematic material, preferably also with a pleochroic dye, for use in Freedericksz effect devices (positive nematic type) in which the molecular arrangement may be changed from the homogeneous texture (OFF state) to the homeotropic texture (ON state) by an electric field;

(iii) as one or more components of a positive material which is a long helical molecular pitch (eg 3 μm) cholesteric (chiral nematic), preferably together also with a pleochroic dye, in cholesteric-to-nematic phase change effect devices (negative contrast type) in which the molecular arrangement may be changed from a scattering focal conic texture (OFF state) to a clear homeotropic texture (ON state) by an electric field;

(iv) as one or more components of a positive nematic material in two frequency switching effect devices (which may be twisted nematic effect devices) in which the dielectric anisotropy of the material may be changed form (at low frequency) positive (OFF state) to negative (ON state) by the application of a high frequency electric field.

(v) As one or more components of a device as described in UK Patent Application No 8317355 Publication No GB 2123163A.

The construction and operation of the above devices and the general kinds of material which are suitable for use in them are themselves known.

Compositions incorporating the compounds of Formula I for use in the above applications may for example contain the following components.

Component A: one or more compounds of Formula I, especially formula Ia, optionally plus one or

more compounds IIa to IIi,

Component B: one or more compounds of Formula IIIa to IIIq;

Component C: one or more compounds of Formula IVa to IVo;

Component D: one or more low melting chiral additives.

For the twisted nematic effect and Freedericksz (positive nematic) effect the following percentages of the various components may be used in the material (the overall sum of the percentages adding to 100%).

Component A: 5 to 95% by weight (typically 5 to 75% by weight)

Component B: 0 to 90% by weight (typically 5 to 25% by weight)

Component C: 0 to 30% by weight (typically 0 to 20% by weight)

Component D: 0 to 5% by weight (typically 0 to 1% by weight)

For the Freedericksz (positive nematic) and phase change (negative contrast type) effects a pleochroic dye forming from ±0.5 to 15% of the overall mixture is preferably added to the liquid crystal material. Suitable dyes are described in published UK Patent Application Nos 2081736A, 208219A and 2093475A. Typically, each dye compound incorporated forms 1 to 3% by weight of the overall mixture.

Liquid crystal mixtures including compounds of Formula I may be formed in a known way, eg simply by heating the constituent compounds together in the correct weight proportion to form an overall isotropic liquid(eg at about 100°C).

To provide a more general example of a mixture embodying the invention at least one compound according to Formula I above may be mixed together with one more compounds in any one or more of the known families listed in table 5 for use in one or more of the applications given above (the actual application(s) depending on the mixture's properties).

In table 5 X is a 1,4 phenylene group, a 4,4′ biphenylyl group, a 2,6 naphthyl group or a trans-1,4-disubstituted cyclohexane ring, and Y is CN, or R′ or halogen or CO.O-X-$Y^1$ where $Y^1$ is CN, or R′ or OR′; where R and R′ are alkyl groups. The compound may alternatively be a derivative of one of these wherein H is replaced by a halogen, eg F, in one of the benzene rings.

Preferably, the compound(s) of Formula I comprises between 5 and 95% by weight of the mixture.

In addition to one or more compounds of formula I, especially of Formula Ia eg the compounds listed in Table 1a, preferred mixtures according to this aspect of the invention contain one or more compounds of formula xxvii and / or xxviii of table 5, particularly where the substituent is -OR′. These preferred mixtures may also contain other liquid crystal compounds, especially those in table 5 having formulae i, iv, v, vi, viii and xxix.

According to the present invention in a second aspect a liquid crystal device includes two dielectric substrates at least one of which is optically transparent, a layer of liquid crystal material sandwiched between the substrates and electrodes on the inner surfaces of the substrates to enable an electric field to be applied across the layer of liquid crystal material to provide an electro-optic effect therein, characterised in that the liquid crystal material consists of or includes a compound according to Formula I above.

The device according to the second aspect may be a twisted nematic effect device, which may or may not be operated in a multiplexed fashion, a cholesteric-to-nematic phase change effect device, a Freedericksz effect device or a two-frequency switching effect device, all constructed in a known manner or any of the other devices mentioned above.

Table 5

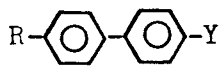 i

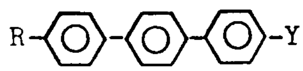 ii

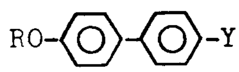 iii

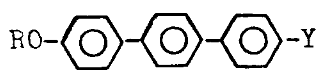 iv

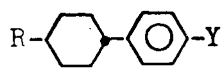 v

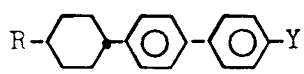 vi

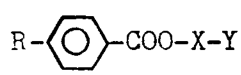 vii

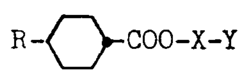 viii

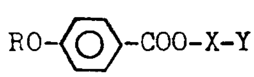 ix

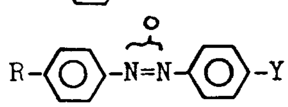 x

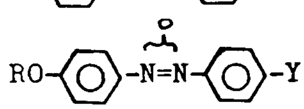 xi

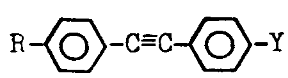 xii

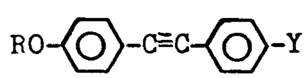 xiii

xiv 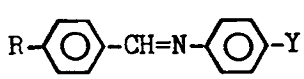

xv 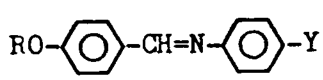

xvi 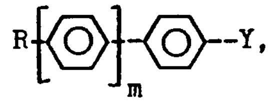, m = 1 or 2

xvii 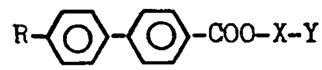

xviii 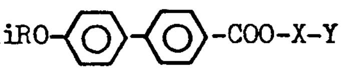

xix 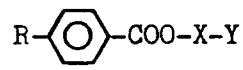

xx 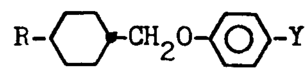

xxi 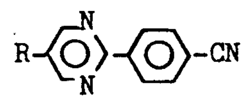

xxii 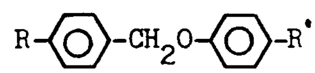

xxiii 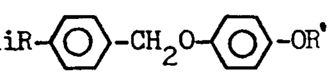

xxiv 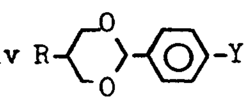

xxv 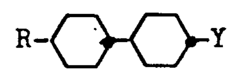

xxvi 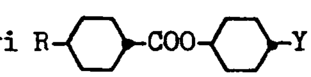

xxvii 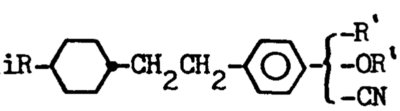

xxviii 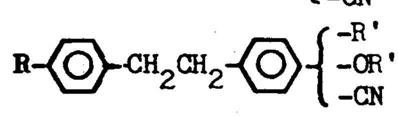

xxix 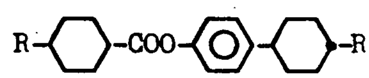

R and R' are preferably $C_{1-8}$ n-alkyl groups.

The various ways in which compounds according to Formula I may be used in these devices are outlined above and will be further apparent to those skilled in the art

Compounds of Formula I may be produced by preparative routes in which the individual steps are known per se, the overall route being new.

The preparation of laterally mono-fluorinated biphenyls of Formula I containing a halogen Q selected from fluorine and chlorine in the 4' - position of the biphenylyl ring and a single ring A (= $A_2$ in Formula I) may be carried out using one of Routes 1 to 4 as shown in Figures 1 to 4 respectively. The starting materials for these Routes may be made by Routes 5 and 6 as shown in Figures 5 and 6.

The preparation of laterally di-fluorinated biphenyls of Formula I containing a halogen Q in the 4′-position of the biphenylyl ring may be carried out for example using Route 7 illustrated in Figure 7 or Route 8 illustrated in Figure 8.

The starting material for Route 8 may be made by Route 9 shown in Figure 9 or Route 10 shown in Figure 10.

The preparation of laterally fluorinated terphenyls of Formula I containing a halogen Q in the 4″-position of the terphenylyl ring may be carried out for example using route 11 illustrated in Figure 11. In figure 11 W, X, Y, Z, $Y^1$ and $Z^1$ are all H or F at least one being F in at least one ring position. Q is F or Cℓ.

Figures 12 and 13 illustrate Routes 12 and 13 respectively for the preparation of $-CF_3$ terminated compounds of Formula I. The cyano-terminated compounds used in Step12a may be produced by cyanation of the corresponding bromo-terminated compounds (eg as produced by Route 9 or Route 10) or produced by Route 14 or Route 15 shown in Figures 14 and 15 respectively where ring A is heterocyclic.

Compounds wherein in Formula I m is 1 may be made by analogous routes which will be apparent to those skilled in the art of synthesis of organic compounds. One such route is route 16 shown in fig. 16. In figs. 1 - 16 R is alkyl or alkoxy and $R_A$ is alkyl.

The invention will now be described by way of example only with reference to the accompanying drawings in which:

Figures 1-16 show preparative routes for the compounds of Formula I.

In this description:

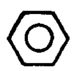 represents a phenyl ring.

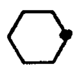 represents trans cyclohexyl.

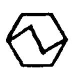 represents bicyclo (2,2,2) octyl.

K, N, S, I represent respectively solid crystal, Nematic liquid crystal, Smectic liquid crystal and Isotropic liquid. All transition temperatures are in °C.

Example 1 Preparation of compound of formula:

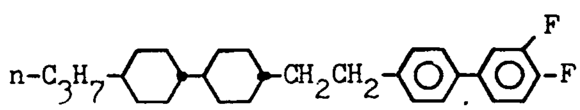

using route 16.

3,4 - Difluorobromobenzene (9.65g) in tetrahydrofuran (10 ml) were added to magnesium (1.23g) in tetrahydrofuran (30 ml) in the absence of atmospheric oxygen and moisture. The reaction mixture was boiled under reflux until all the magnesium had dissolved. The mixture was cooled to room temperature and was then added, under ice cooling, to a solution of zinc chloride (3.4g) in tetrahydrofuran (25 ml). After 30 minutes dichloro-bistriphenylphosphine-nickel (II) (0.33g) was added, followed by a solution of p-[trans-4-(trans-4-n-propylcyclohexyl)-cyclohexylethyl]-bromobenzene (20.5g) in tetrahydrofuran. After 24 hours standing at room temperature the reaction mixture was worked up in the usual way to yield 4-[trans-4-(trans-4-n-propylcyclohexyl)-cyclohexylethyl]-$3^1,4^1$-difluorobiphenyl. Mpt 82°C, clearing point 216°C.

This compound had an extrapolated viscosity of 28.0 cp at 20°C. This is quite low and thus the compound may be used to reduce the viscosity whilst raising the clearing point of a liquid crystal composition in which it is contained.

By a completely analogous route the following compounds were prepared, of general formula

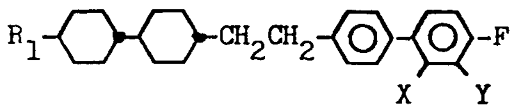

Table 6

| $R_1$ | X | Y | X | Y |
|---|---|---|---|---|
| methyl | H | F | F | H |
| ethyl | " | " | " | " |
| n-butyl | " | " | " | " |
| n-pentyl | " | " | " | " |
| n-hexyl | " | " | " | " |
| n-heptyl | " | " | " | " |
| n-octyl | " | " | " | " |
| n-nonyl | " | " | " | " |
| n-decyl | " | " | " | " |
| n-undecyl | " | " | " | " |
| n-dodecyl | " | " | " | " |
| 2-methylbutyl (+), (-) and (±) | " | " | " | " |
| 3-methylpentyl (+), (-) and (±) | " | " | " | " |
| 4-methylhexyl (+), (-) and (±) | " | " | " | " |
| methoxy | " | " | " | " |
| ethoxy | " | " | " | " |
| n-propyloxy | " | " | " | " |
| n-butyloxy | " | " | " | " |
| n-pentyloxy | " | " | " | " |
| n-hexyloxy | " | " | " | " |
| n-heptyloxy | " | " | " | " |
| n-octyloxy | " | " | " | " |
| n-nonyloxy | " | " | " | " |
| n-decyloxy | " | " | " | " |
| n-undecyloxy | " | " | " | " |
| 2-methylbutyloxy (+), (-) and (±) | " | " | " | " |
| 3-methylheptyloxy (+), (-) and (±) | " | " | " | " |
| 4-methylhexyloxy (+), (-) and (±) | " | " | " | " |

Example 2 Preparation of compounds of general formula:

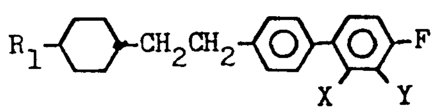

where one of Z and Y is F and the other H.

These compounds were prepared by a method analogous to that of example 1, but using p-[trans-4-alkyl or alkoxy cyclohexylethyl]-bromobenzeneso that only one cyclohexyl ring is introduced into the molecule.

The properties of some of these compounds, where X is hydrogen and Y is fluorine are listed below:

| $R_1$ | K-N ° C | N-I ° C |
|---|---|---|
| ethyl | 35.2 | 61.6 |
| n-propyl | 64.1 | 88.7 |
| n-butyl | 50.6 | 87.0 |
| n-pentyl | 74.8 | 95.9 |
| n-hexyl | 48.2 | 92.0 |

By a completely analogous route to that of example 2, the compounds listed in table 7 were prepared.

Table 7

| $R_1$ | X | Y | X | Y |
|---|---|---|---|---|
| methyl | H | F | H | F |
| ethyl | F | H | | |
| n-propyl | F | H | | |
| n-butyl | F | H | | |
| n-pentyl | F | H | | |
| n-hexyl | F | H | | |
| n-octyl | F | H | H | F |
| n-nonyl | " | " | " | " |
| n-decyl | " | " | " | " |
| n-undecyl | " | " | " | " |
| n-dodecyl | " | " | " | " |
| 2-methylbutyl (+), (-) and (±) | " | " | " | " |
| 3-methylpentyl (+), (-) and (±) | " | " | " | " |
| 4-methylhexyl (+), (-) and (±) | " | " | " | " |
| 5-methylheptyl (+), (-) and (±) | " | " | " | " |
| methoxy | " | " | " | " |
| ethoxy | " | " | " | " |
| n-propyloxy | " | " | " | " |
| n-butyloxy | " | " | " | " |
| n-pentyloxy | " | " | " | " |
| n-hexyloxy | " | " | " | " |
| n-heptyloxy | " | " | " | " |
| n-octyloxy | " | " | " | " |
| n-nonyloxy | " | " | " | " |
| n-decyloxy | " | " | " | " |
| 2-methylbutyloxy (+), (-) and (±) | " | " | " | " |
| 3-methylpentyloxy (+), (-) and (±) | " | " | " | " |
| 4-methylhexyloxy (+), (-) and (±) | " | " | " | " |
| n-undecyloxy | " | " | " | " |
| n-dodecyloxy | " | " | " | " |

The compounds referred to above may for example be used in the devices particularly described with reference to the drawings in published UK Patent Application No 2121406A, the above compounds (either alone or in mixture form) replacing the compound 1-(4-cyano-phenyl)-2-(4-n-pentylphenyl)ethane in mixtures 1, 2 and 3 specified in that application.

Example 3. Nematic liquid crystal mixtures containing compounds of formula I.

Mixture A

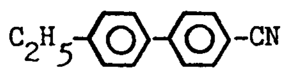 15 wt. %

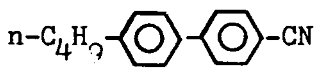 13 wt. %

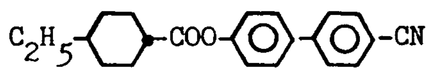 12 wt. %

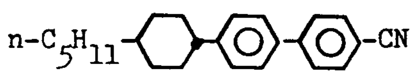 10 wt. %

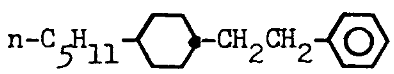 10 wt. %

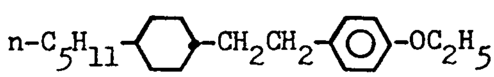 20 wt. %

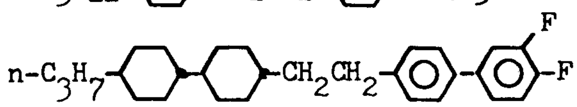 20 wt. %

Mixture B

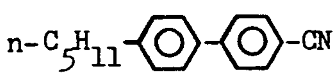 29 wt. %

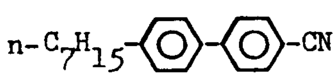 17 wt. %

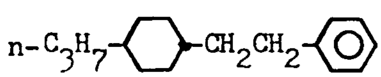 11 wt. %

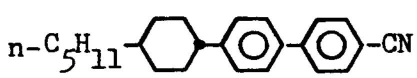 8 wt. %

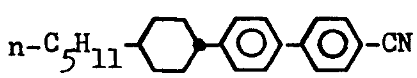 10 wt. %

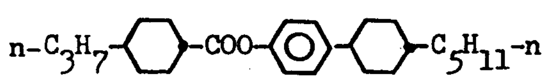 10 wt. %

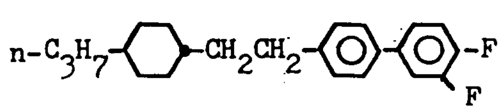 15 wt. %

## Claims

**Claims for the following Contracting States : AT, BE, FR, GB, IT, NL, SE**

**1.** A compound suitable for use in liquid crystal materials having a formula:

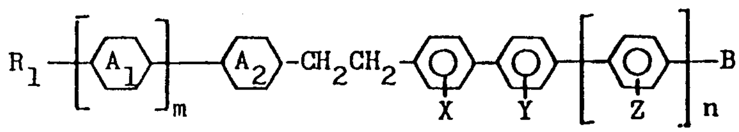

wherein:
m represents 0 or 1;
n represents 0 or 1;
$R_1$ represents hydrogen or an alkyl, alkoxy, alkoxy substituted alkyl, alkanoyl, alkanoyloxy, alkoxycarbonyl or alkoxycarbonyloxy group;
each of

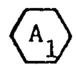 and 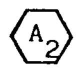

represents a group selected from 1,4-disubstituted cyclohexane with the substituents in the trans-configuration; 1,4-disubstituted bicyclo (2,2,2) octane; 2,5-disubstituted 1,3-dioxan with the substituents in the trans-configuration; 1,4-disubstituted benzene optionally substituted with fluorine, eg in the 2 and/or 3 positions, 2,5-disubstituted 1,3 pyrimidine and 3,6-disubstituted 1,2 pyridazine;

each of X, Y and Z represent hydrogen and fluorine in the 2,3,5 and 6 benzene ring positions provided that at least one of X, Y and Z represents fluorine in at least one ring position; and

B represents a terminal group selected from:
fluoro, chloro and $CF_3$.

**2.** A compound according to claim 1, having a formula:

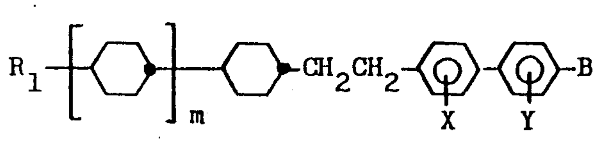

where at least one of X and Y is fluorine.

**3.** A compound according to claim 1 or 2 characterised in that $R_1$ is $C_{1-12}$ alkyl or alkoxy and B is fluorine.

**4.** A compound according to claim 3 having a formula

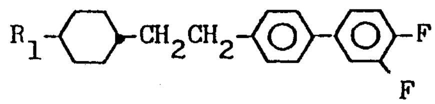

**5.** A compound according to claim 4 characterised in that $R_1$ contains 2-6 carbon atoms.

**6.** A compound according to claim 3 having a formula

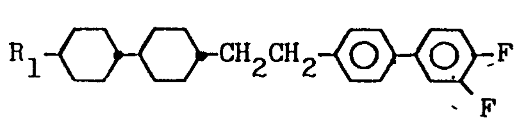

**7.** A compound according to claim 6 characterised in that $R_1$ is n-propyl.

**8.** A liquid crystal material having at least two components characterised in that at least one component is a compound as claimed in any one of the preceding claims.

**9.** A liquid crystal material according to claim 8 characterised in that it contains a compound of formula

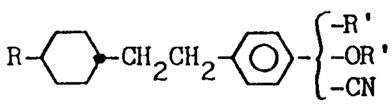 or 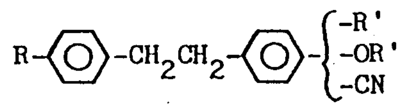

where R and R' are alkyl groups.

**10.** A liquid crystal electro-optical display device characterised in that it incorporates a material as claimed in claim 8 or 9.

**Claims for the following Contracting States : CH, DE, LI**

**1.** A compound suitable for use in liquid crystal materials characterised by a formula:

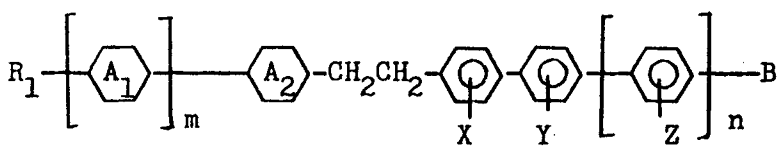

wherein:
m represents 0 or 1;
n represents 0 or 1;
$R_1$ represents hydrogen or an alkyl, alkoxy, alkoxy substituted alkyl, alkanoyl, alkanoyloxy, alkoxycarbonyl or alkoxycarbonyloxy group;

each of

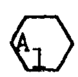 and 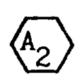

represents a group selected from 1,4-disubstituted cyclohexane with the substituents in the trans-configuration; 1,4-disubstituted bicyclo (2,2,2) octane; 2,5-dissubstituted 1,3-dioxan with the substituents in the trans-configuration; 1,4-dissubstituted benzene optionally substituted with fluorine, eg in the 2 and/or 3 positions, 2,5-disubstituted 1,3 pyrimidine and 3,6-dissubstituted 1,2 pyridazine;

each of X, Y and Z represents hydrogen and fluorine in the 2,3,5 and 6 benzene ring positions provided that at least one of X, Y and Z represents fluorine in at least one ring position; and

B represents a terminal group selected from:

fluoro, chloro and $CF_3$ ;

with the proviso that if rings

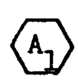 and 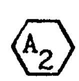

are cyclohexane, and X is hydrogen and Y is fluorine in the 3- position and B is fluorine and $R_1$ is alkyl, then $R_1$ is selected from $C_{4-12}$ chiral alkyl or if m and n are both 0 then $R_1$ is selected from n-butyl, n-pentyl, n-hexyl and n-octyl, or if m is l and n is 0 then $R_1$ is selected from n-propyl, n-butyl, n-pentyl, n-hexyl and n-octyl.

**2.** A compound according to claim 1 characterised by a formula:

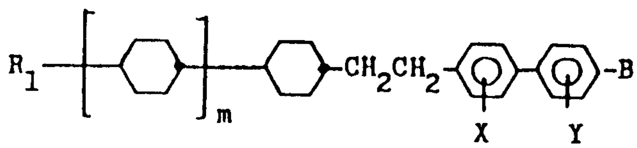

wherein at least one X or Y is fluorine.

**3.** A compound according to claim 2 characterised in that, $R_1$ is $C_{1-12}$ alkyl or alkoxy, B is fluorine and one of X or Y is fluorine, and provided that if Y is fluorine in the 3- position and $R_1$ is alkyl then either $R_1$ is $C_{4-12}$ chiral alkyl, or if m is 0 then $R_1$ is selected from n-butyl, n-pentyl, n-hexyl and n-octyl, or if m is 1 then $R_1$ is selected from n-propyl, n-butyl, n-pentyl, n-hexyl and n-octyl-.

**4.** A compound according to claim 2 characterised by a formula:

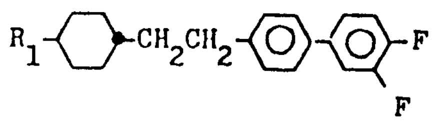

wherein $R_1$ is selected from n-butyl, n-pentyl, n-hexyl and n-octyl.

**5.** A compound according to claim 2 characterised by a formula:

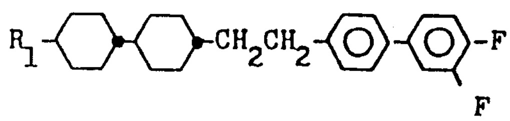

wherein $R_1$ is selected from N-propyl, n-butyl, n-pentyl, n-hexyl and n-octyl.

**6.** A compound according to claim 5 characterised in that $R_1$ is n-propyl.

**7.** A liquid crystal material having at least two components characterised in that at least one component is a compound as claimed in any one of the preceding claims.

**8.** A liquid crystal material according to claim 7 characterised in that it contains a compound of formula

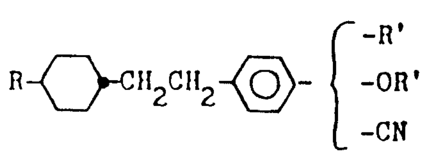

or

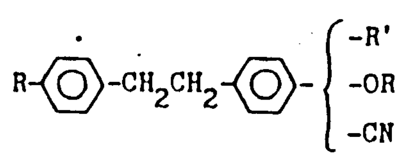

where R and R' are alkyl groups.

**9.** A liquid crystal electro-optical display device characterised in that it incorporates a material as claimed in claim 7 or 8

**Revendications**

**Revendications pour les Etats contractants suivants : AT, BE, FR, GB, IT, NL, SE**

**1.** Composé convenant pour servir dans des matières à propriétés de cristaux liquides et ayant pour formule :

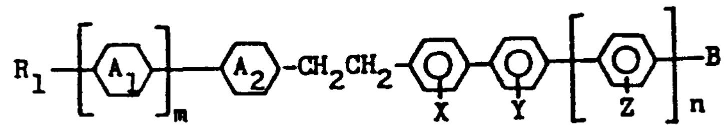

dans laquelle

m représente un nombre valant 0 ou 1 ;

n représente un nombre valant 0 ou 1 ;

$R_1$ représente un atome d'hydrogène ou un groupe alkyle, alcoxy, alkyle à substituant alcoxy, alcanoyle, alcanoyloxy, alcoxycarbonyle ou alcoxycarbonyloxy ;

chacun des symboles

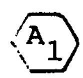 et 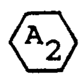

représente un groupe choisi parmi un groupe cyclohexane disubstitué en 1,4, et ayant les substituants en configuration trans ; un groupe bicyclo(2,2,2)octane disubstitué en 1,4 ; un groupe 1,3-dioxanne disubstitué en 2,5, et ayant les substituants en configuration trans ; un noyau benzénique disubstitué en 1,4 et éventuellement substitué par du fluor, par exemple sur les positions 2 et/ou 3, un groupe 1,3-pyrimidine disubstitué en 2,5 et un groupe 1,2-pyridazine disubstitué en 3,6 ;

chacun des symboles X, Y et Z représente un atome d'hydrogène et de fluor sur les positions 2, 3, 5 et 6 du noyau benzénique, à la condition qu'au moins l'un des symboles X, Y et Z représente un atome de fluor fixé sur une au moins des positions de noyau ; et

B représente un groupe terminal choisi parmi un groupe fluoro, chloro et $CF_3$.

**2.** Composé selon la revendication 1, ayant pour formule

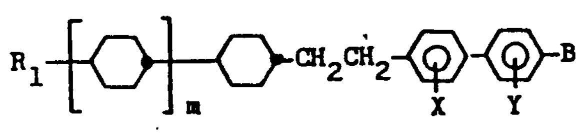

dans laquelle au moins l'un des symboles X et Y représente un atome de fluor.

**3.** Composé selon la revendication 1 ou 2, caractérisé en ce que le symbole $R_1$ représente un groupe alkyle ou alcoxy en $C_1$ à $C_{12}$, et B représente un atome de fluor.

**4.** Composé selon la revendication 3, ayant pour formule :

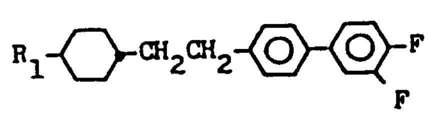

**5.** Composé selon la revendication 4, caractérisé en ce que le groupe $R_1$ contient 2 à 6 atomes de carbone.

**6.** Composé selon la revendication 3, ayant pour formule

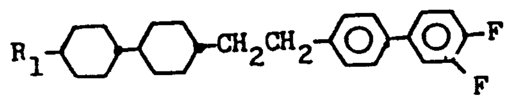

7. Composé selon la revendication 6, caractérisé en ce que $R_1$ représente un groupe n-propyle.

8. Matière à propriétés de cristaux liquides, comportant au moins deux composants et caractérisée en ce qu'au moins un composant est un composé tel que revendiqué dans l'une quelconque des revendications précédentes.

9. Matière à propriétés de cristaux liquides selon la revendication 8, caractérisée en ce qu'elle contient un composé de formule

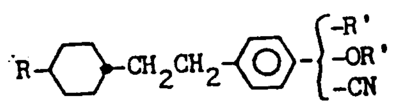

ou

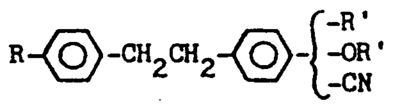

formules dans lesquelles R et R' représentent chacun un groupe alkyle.

10. Dispositif d'affichage électro-optique à cristaux liquides, caractérisé en ce qu'il incorpore une matière telle que revendiquée aux revendications 8 ou 9.

**Revendications pour les Etats contractants suivants : CH, DE, LI**

1. Composé convenant pour servir dans des matières à propriétés de cristaux liquides, caractérisé par une formule

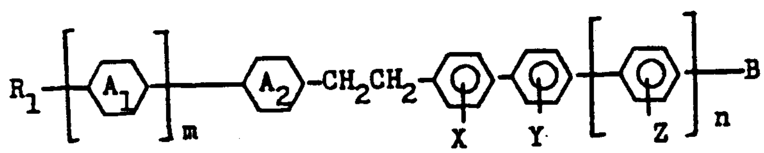

dans laquelle
m représente un nombre valant 0 ou 1 ;
n représente un nombre valant 0 ou 1 ;
$R_1$ représente un atome d'hydrogène ou un groupe alkyle, alcoxy, alkyle à substituant alcoxy, alcanoyle, alcanoyloxy, alcoxycarbonyle ou alcoxycarbonyloxy ;
chacun des symboles

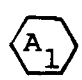

et

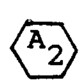

représente un groupe choisi parmi un groupe cyclohexane disubstitué en 1,4, les substituants étant en configuration trans ; un groupe bicyclo(2,2,2)octane disubstitué en 1,4 ; 1,3-dioxanne disubstitué en 2,5, dont les substituants sont en configuration trans ; un noyau benzénique disubstitué en 1,4 et éventuellement substitué par du fluor, par exemple sur les positions 2 et/ou 3, un groupe 1,3 pyrimidine disubstitué en 2,5 et un groupe 1,2-pyridazine disubstitué en 3,6 ;
chacun des symboles X, Y et Z représente un atome d'hydrogène et de fluor sur les positions 2, 3, 5 et 6 du noyau benzénique, à la condition qu'au moins l'un des symboles X, Y et Z représente du fluor sur une au moins des positions de noyau ; et

B représente un groupe terminal choisi parmi :
un groupe fluoro, chloro et $CF_3$; à la condition que si les noyaux

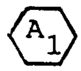 et 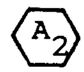

représentent chacun du cyclohexane, et si X représente un atome d'hydrogène et Y représente un atome de fluor sur la position 3 et si B représente un atome de fluor et $R_1$ représente un groupe alkyle, le symbole $R_1$ est alors choisi parmi un groupe alkyle chiral en $C_4$ à $C_{12}$, ou bien si m et n sont chacun nuls, le symbole $R_1$ est alors choisi parmi un groupe n-butyle, n-pentyle, n-hexyle et n-octyle, ou bien, si m vaut 1 et si n est nul, $R_1$ est alors choisi parmi un groupe n-propyle, n-butyle, n-pentyle, n-hexyle et n-octyle.

**2.** Composé selon la revendication 1, caractérisé par une formule

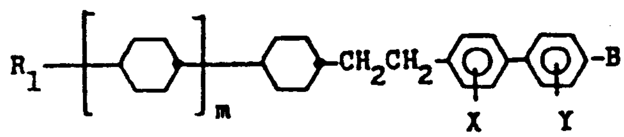

dans laquelle l'un au moins des symboles X ou Y représente le fluor.

**3.** Composé selon la revendication 2, caractérisé en ce que $R_1$ représente un groupe alkyle ou alcoxy en $C_1$ à $C_{12}$, a représente un atome de fluor et l'un des symboles X ou Y représente un atome de fluor, à la condition que, si Y représente un atome de fluor en position 3 et si $R_1$ représente un groupe alkyle, ou bien $R_1$ représente alors un groupe alkyle chiral en $C_4$ à $C_{12}$ ou bien, si m est nul, $R_1$ est alors choisi parmi un groupe n-butyle, n-pentyle, n-hexyle et n-octyle, ou bien si m vaut 1, le groupe représenté par $R_1$ est choisi parmi un groupe n-propyle, n-butyle, n-pentyle, n-hexyle et n-octyle.

**4.** Composé selon la revendication 2, caractérisé par une formule

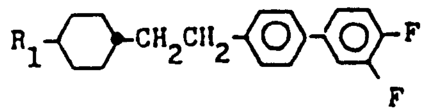

dans laquelle le groupe représenté par le symbole $R_1$ est choisi parmi un groupe n-butyle, n-pentyle, n-hexyle et n-octyle.

**5.** Composé selon la revendication 2, caractérisé par une formule

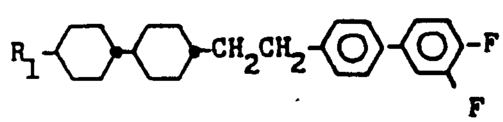

dans laquelle le groupe représenté par le symbole $R_1$ est choisi parmi un groupe n-propyle, n-butyle, n-pentyle, n-hexyle et n-octyle.

**6.** Composé selon la revendication 5, caractérisé en ce que $R_1$ représente un groupe n-propyle.

**7.** Matière à propriétés de cristaux liquides, comportant au moins deux composants, matière caractérisée en ce qu'au moins un composant est un composé tel que revendiqué dans l'une quelconque des

revendications précédentes.

**8.** Matière à propriétés de cristaux liquides selon la revendication 7, caractérisée en ce qu'elle contient un composé de formule

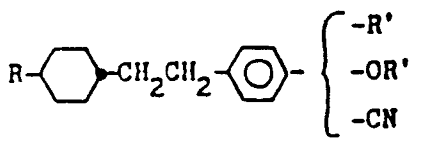

ou

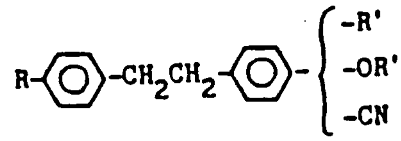

dans laquelle les symboles R et R' représentent des groupes alkyles.

**9.** Dispositif d'affichage électro-optique à cristaux liquides, caractérisé en ce qu'il incorpore une matière telle que revendiquée à la revendication 7 ou 8.

**Patentansprüche**
**Patentansprüche für folgende Vertragsstaaten : AT, BE, FR, GB, IT, NL, SE**

**1.** Verbindungen zur Verwendung in Flüssigkristallmaterialien,

gekennzeichnet durch die Formel

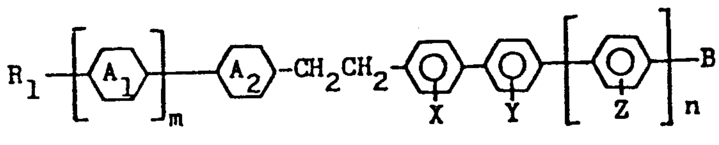

,

in der bedeuten:

n 0 oder 1;
n 0 oder 1;
$R_1$ Wasserstoff, Alkyl, Alkoxy, alkoxysubstituiertes Alkyl, Alkanoyl, Alkanoyloxy, Alkoxycarbonyl oder Alkoxycarbonyloxy;

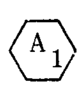

und

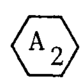

jeweils eine Gruppe, die ausgewählt ist unter 1,4-disubstituiertem Cyclohexan mit den Substituenten in trans-Konfiguration, 1,4-disubstituiertes Bicyclo-(2.2.2)-octan, 2,5-disubstituiertes 1,3-Dioxan mit den Substituenten in trans-Konfiguration, 1,4-disubstituiertes Benzol, das wahlweise mit Fluor substituiert ist, z.B. in den Positionen 2 und/oder 3, 2,5-disubstituiertes 1,3-Pyrimidin und 3,6-disubstituiertes 1,2-Pyridazin;

X, Y und Z jeweils Wasserstoff oder Fluor in den Positionen 2, 3, 5 und 6 des Benzolrings, mit der Maßgabe, daß zumindest einer der Substituenten X, Y und Z Fluor in mindestens einer Ringposition darstellt,

und

B eine Endgruppe, die ausgewählt ist unter Fluor, Chlor und $CF_3$.

**2.** Verbindungen nach Anspruch 1 der Formel

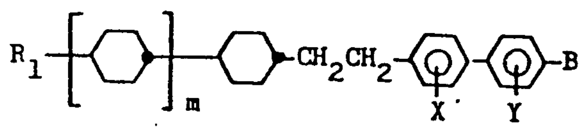 ,

in der mindestens einer der Substituenten X und Y Fluor bedeutet.

**3.** Verbindungen nach Anspruch 1 oder 2, dadurch gekennzeichnet, daß $R_1$ $C_{1-12}$-Alkyl oder -alkoxy und B Fluor bedeuten.

**4.** Verbindungen nach Anspruch 3 der Formel

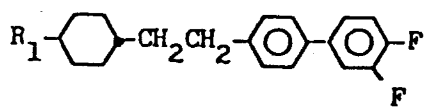 .

**5.** Verbindungen nach Anspruch 4, dadurch gekennzeichnet, daß $R_1$ 2 bis 6 Kohlenstoffatome enthält.

**6.** Verbindungen nach Anspruch 3 der Formel

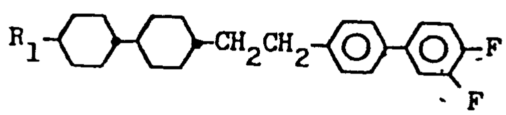 .

**7.** Verbindung nach Anspruch 6, dadurch gekennzeichnet, daß $R_1$ n-Propyl ist.

**8.** Flüssigkristallmaterialien mit mindestens zwei Komponenten, dadurch gekennzeichnet, daß mindestens eine Komponente eine Verbindung nach einem der vorhergehenden Ansprüche ist.

**9.** Flüssigkristallmaterialien nach Anspruch 8, dadurch gekennzeichnet, daß sie eine Verbindung der Formeln

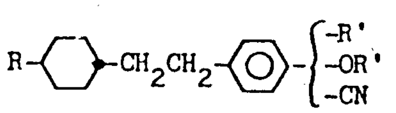 oder 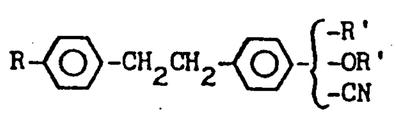

enthalten, worin R und R' Alkylgruppen bedeuten.

**10.** Elektrooptische Flüssigkristall-Anzeigevorrichtungen, dadurch gekennzeichnet, daß sie ein Flüssigkristallmaterial nach Anspruch 8 oder 9 enthalten.

**Patentansprüche für folgende Vertragsstaaten : CH, DE, LI**

**1.** Verbindungen zur Verwendung in Flüssigkristallmaterialien,

gekennzeichnet durch die Formel

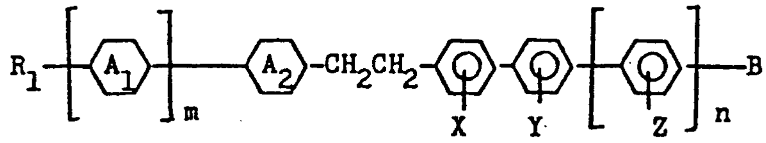

,

in der bedeuten:

m 0 oder 1;
n 0 oder 1;
$R_1$ Wasserstoff, Alkyl, Alkoxy, alkoxysubstituiertes Alkyl, Alkanoyl, Alkanoyloxy, Alkoxycarbonyl oder Alkoxycarbonyloxy;

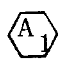 und 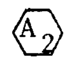

jeweils eine Gruppe, die ausgewählt ist unter 1,4-disubstituiertem Cyclohexan mit den Substituenten in trans-Konfiguration, 1,4-disubstituiertes Bicyclo-(2.2.2)-octan, 2,5-disubstituiertes 1,3-Dioxan mit den Substituenten in trans-Konfiguration, 1,4-disubstituiertes Benzol, das wahlweise mit Fluor substituiert ist, z.B. in den Positionen 2 und/oder 3, 2,5-disubstituiertes 1,3-Pyrimidin und 3,6-disubstituiertes 1,2-Pyridazin;

X, Y und Z jeweils Wasserstoff oder Fluor in den Positionen 2, 3, 5 und 6 des Benzolrings, mit der Maßgabe, daß zumindest einer der Substituenten X, Y und Z Fluor in mindestens einer Ringposition darstellt,

und

B eine Endgruppe, die ausgewählt ist unter Fluor, Chlor und $CF_3$, mit der Maßgabe, daß dann, wenn die Ringe

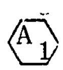 und 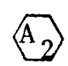

Cyclohexan, X Wasserstoff, Y Fluor in 3-Position, B Fluor und $R_1$ Alkyl bedeuten, $R_1$ unter den chiralen $C_{4-12}$-Alkylgruppen ausgewählt ist, oder dann, wenn m und 1 beide gleich 0 sind, $R_1$ unter n-Butyl, n-Pentyl, n-Hexyl und n-Octyl ausgewählt ist, oder dann, wenn m gleich 1 und n gleich 0 sind, $R_1$ unter n-Propyl, n-Butyl, n-Pentyl, n-Hexyl und n-Octyl ausgewählt ist.

**2.** Verbindungen nach Anspruch 1, gekennzeichnet durch die Formel

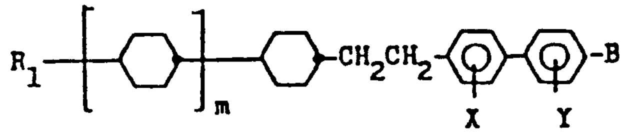

,

in der mindestens einer der Substituenten X und Y Fluor bedeutet.

**3.** Verbindungen nach Anspruch 2, dadurch gekennzeichnet, daß $R_1$ $C_{1-12}$-Alkyl oder -alkoxy, B Fluor und einer der Substituenten X und Y Fluor bedeuten, mit der Maßgabe, daß dann, wenn Y Fluor in 3-Position und $R_1$ Alkyl bedeuten, $R_1$ jeweils chirales $C_{4-12}$-Alkyl darstellt, oder dann, wenn m gleich 0 ist, $R_1$ unter n-Butyl, n-Pentyl, n-Hexyl und n-Octyl ausgewählt ist, oder dann, wenn m gleich 1 ist, $R_1$ unter n-Propyl, n-Butyl, n-Pentyl, n-Hexyl und n-Octyl ausgewählt ist.

**4.** Verbindungen nach Anspruch 2, gekennzeichnet durch die Formel

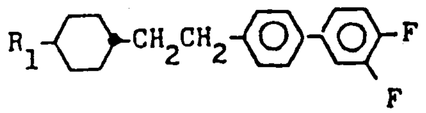
,

in der $R_1$ unter n-Butyl, n-Pentyl, n-Hexyl und n-Octyl ausgewählt ist.

**5.** Verbindungen nach Anspruch 2, gekennzeichnet durch die Formel

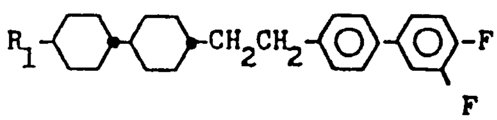
,

in der $R_1$ unter n-Propyl, n-Butyl, n-Pentyl, n-Hexyl und n-Octyl ausgewählt ist.

**6.** Verbindungen nach Anspruch 5, dadurch gekennzeichnet, daß $R_1$ n-Propyl ist.

**7.** Flüssigkristallmaterialien mit mindestens zwei Komponenten, dadurch gekennzeichnet, daß mindestens eine Komponente eine Verbindung nach einem der vorhergehenden Ansprüche ist.

**8.** Ein flüssigkristallines Material nach Anspruch 7, dadurch gekennszeichnet, daß es eine Verbindung der Formel

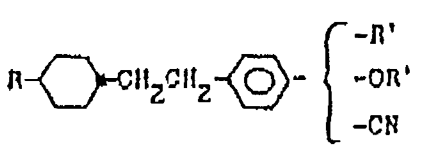
or
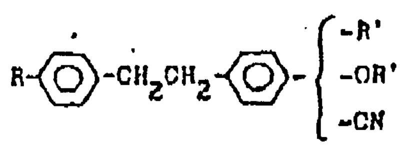

enthält, worin R und R' Alkylreste sind.

**9.** Eine flüssigkristalline, elektrooptische Anzeigeeinrichtung, dadurch gekennzeichnet, daß sie ein wie im Anspruch 7 oder 8 beschriebenes Material beinhaltet.

Route 1

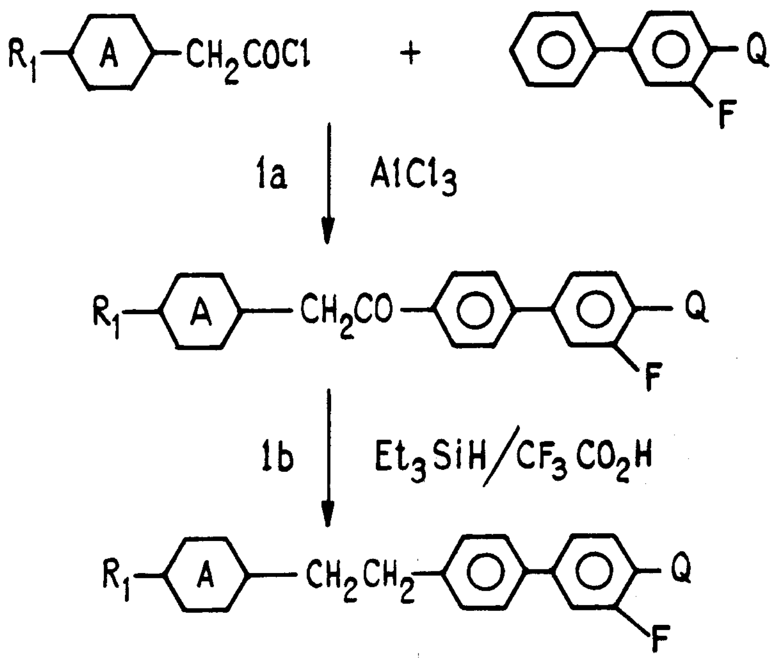


FIG. 1

Route 2

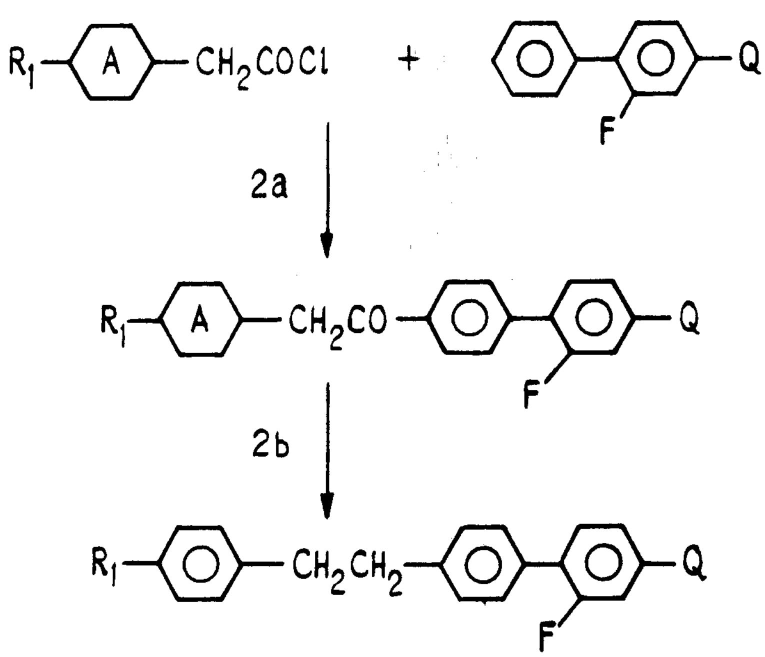


FIG. 2

Route 3

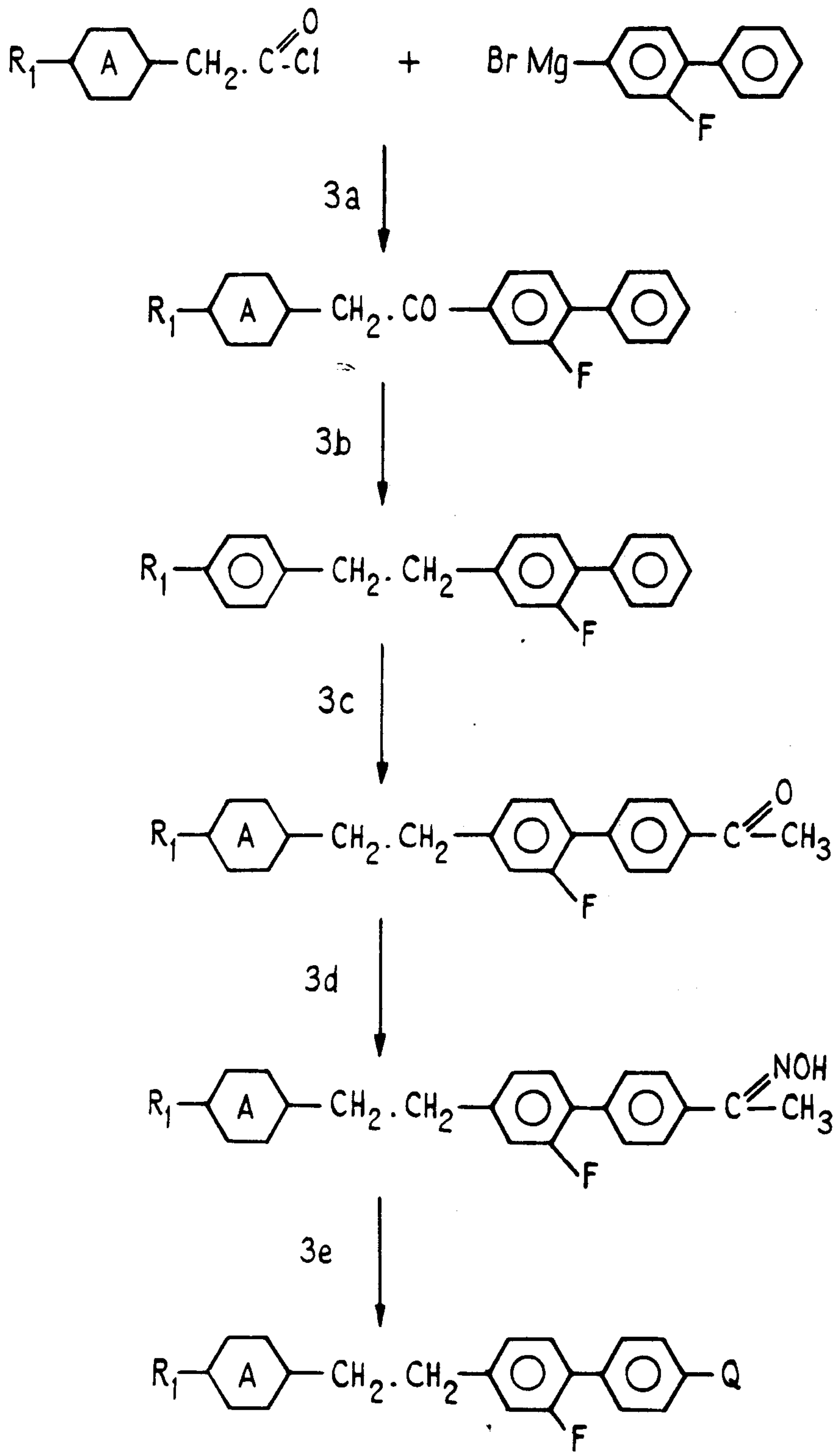


FIG.3

Route 4

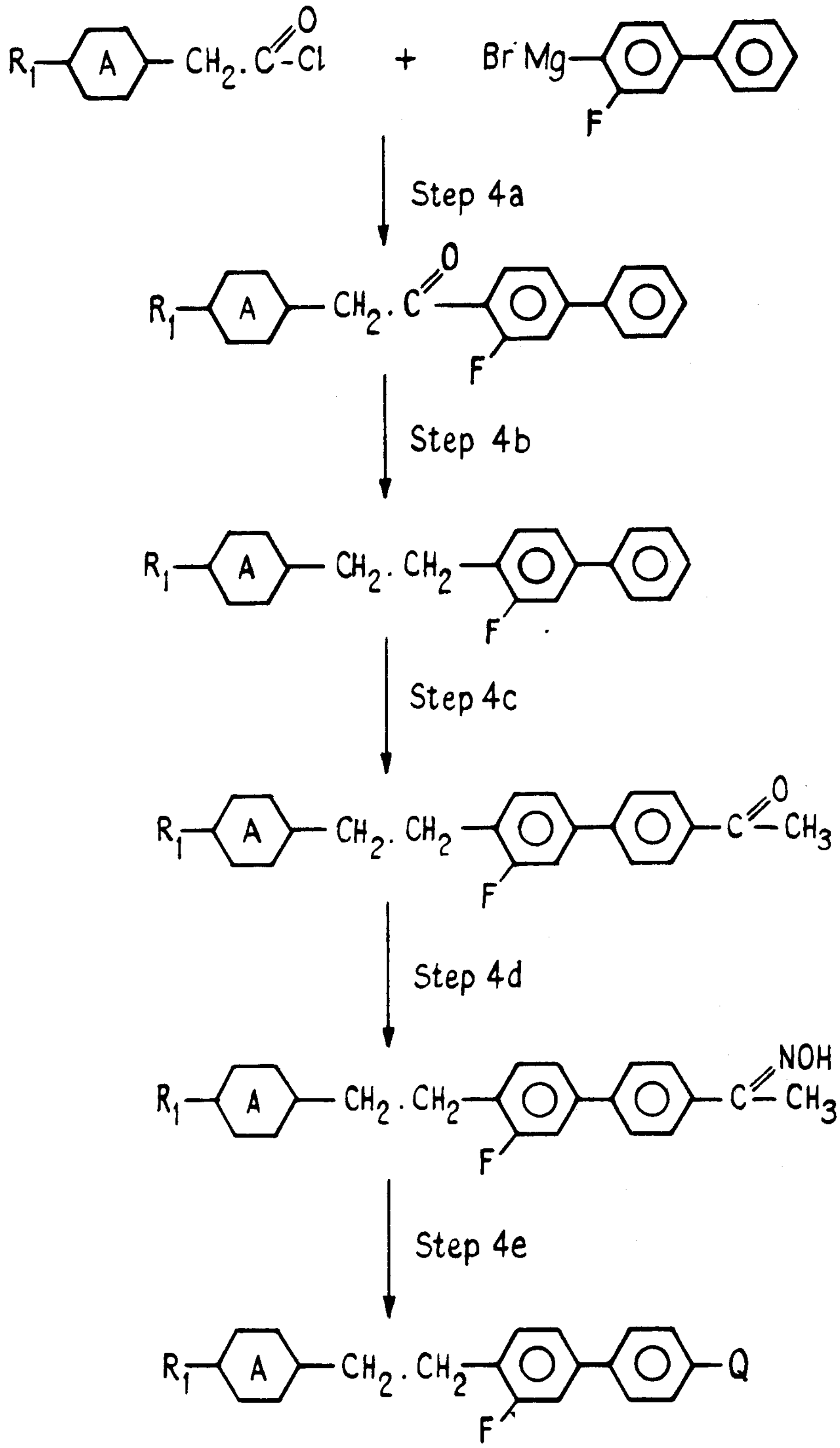


FIG.4

Route 5

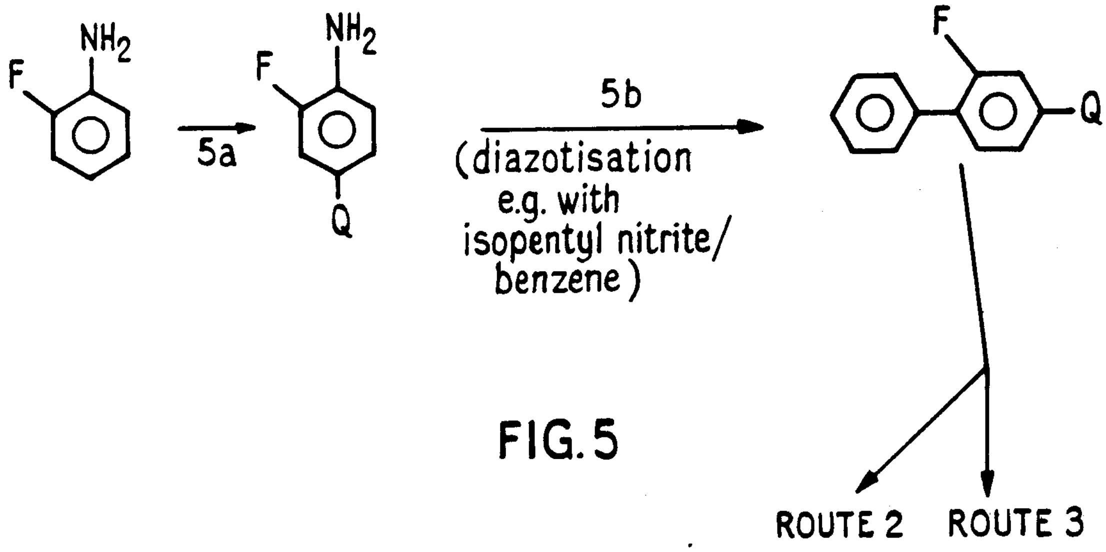


FIG. 5

Route 6

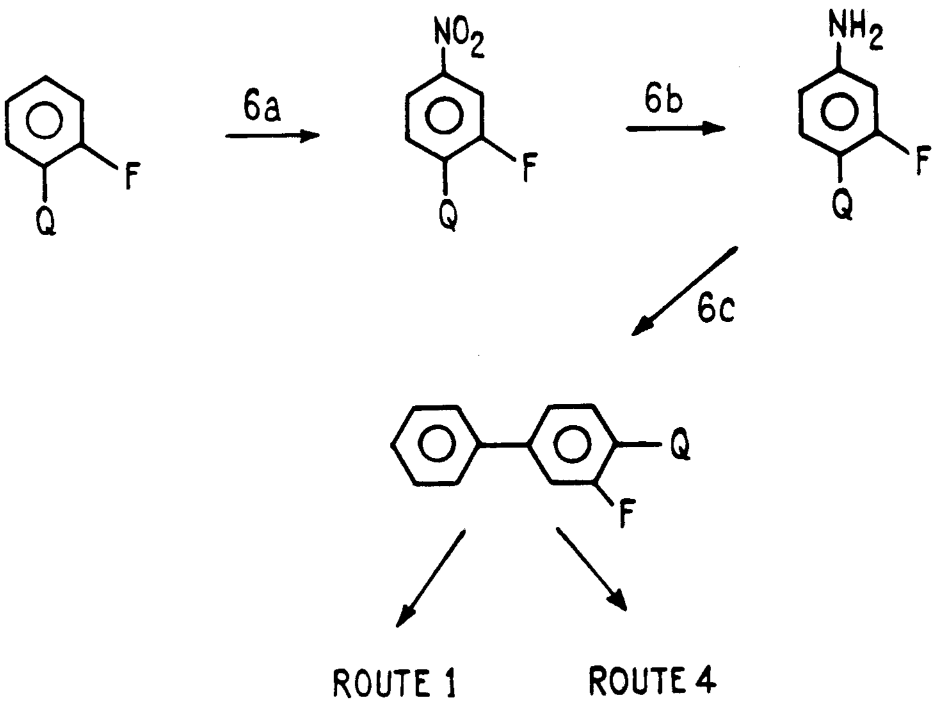


FIG. 6

Route 7

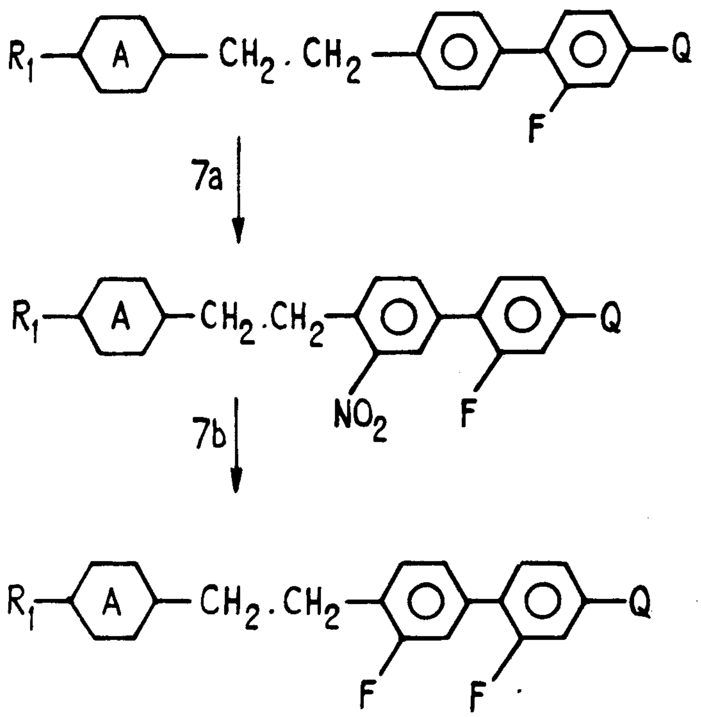


FIG.7

Route 8

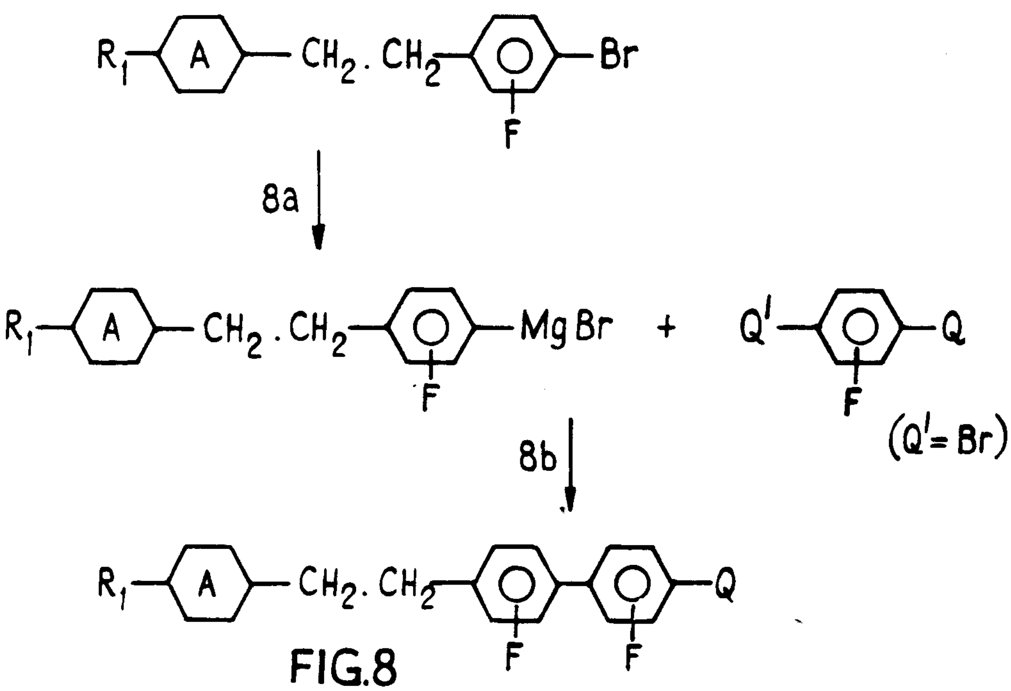


FIG.8

Route 9

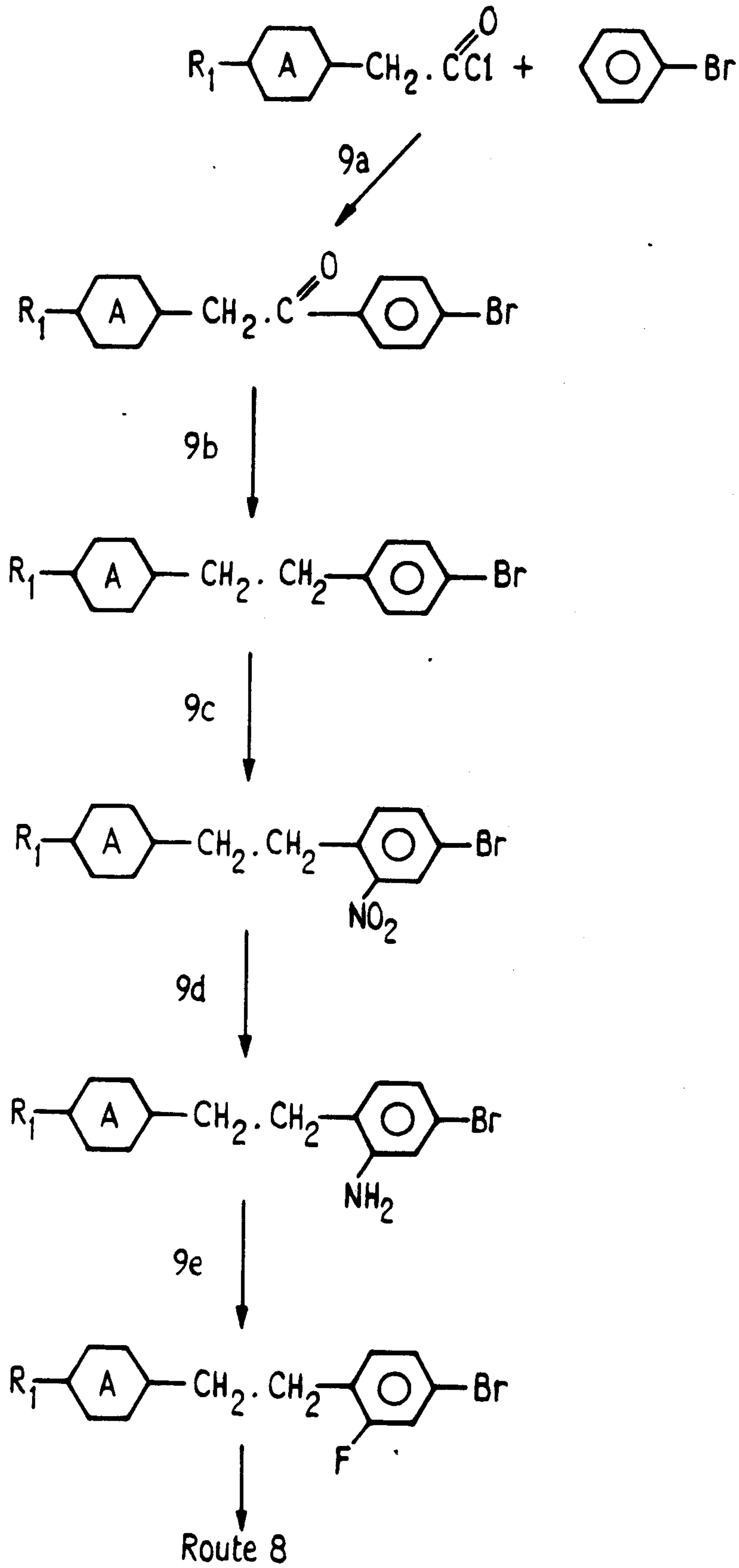


FIG.9

Route 10

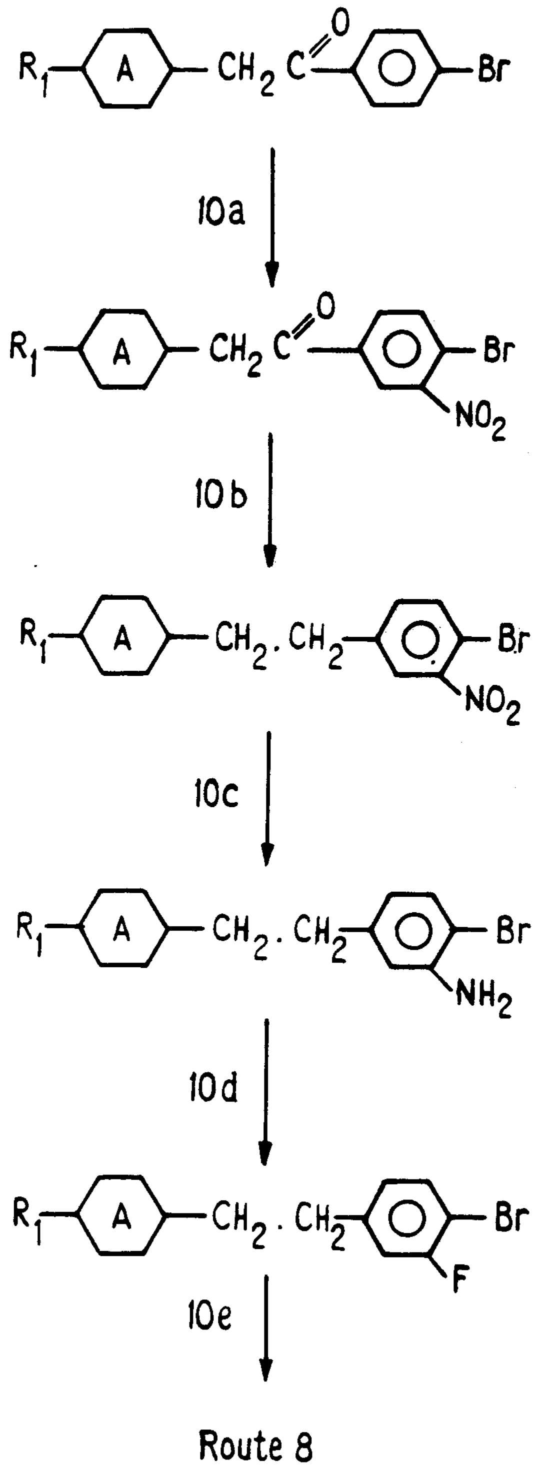


FIG. 10

Route 11

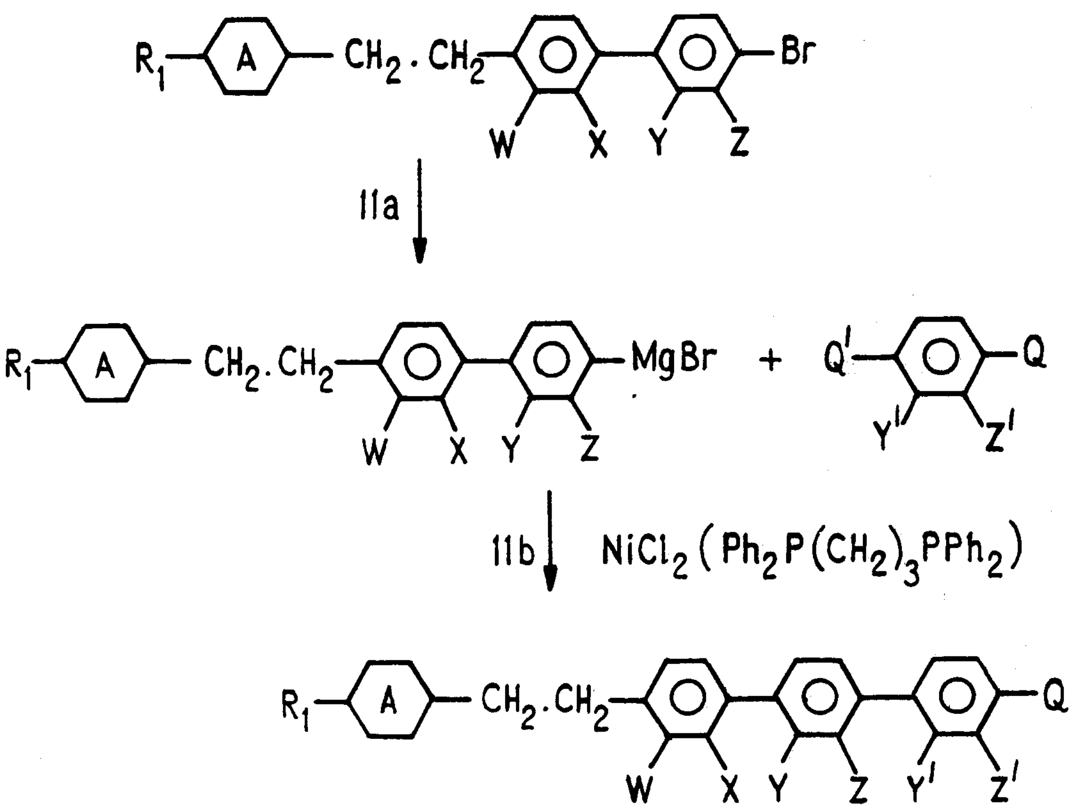


FIG.11

Route 12

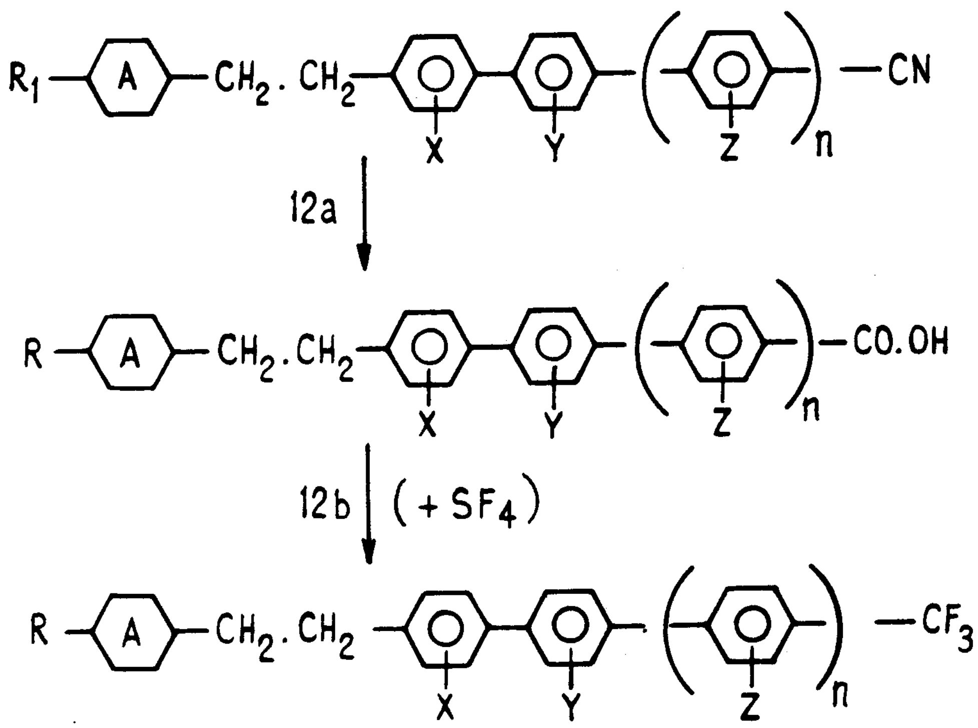


FIG. 12

Route 13

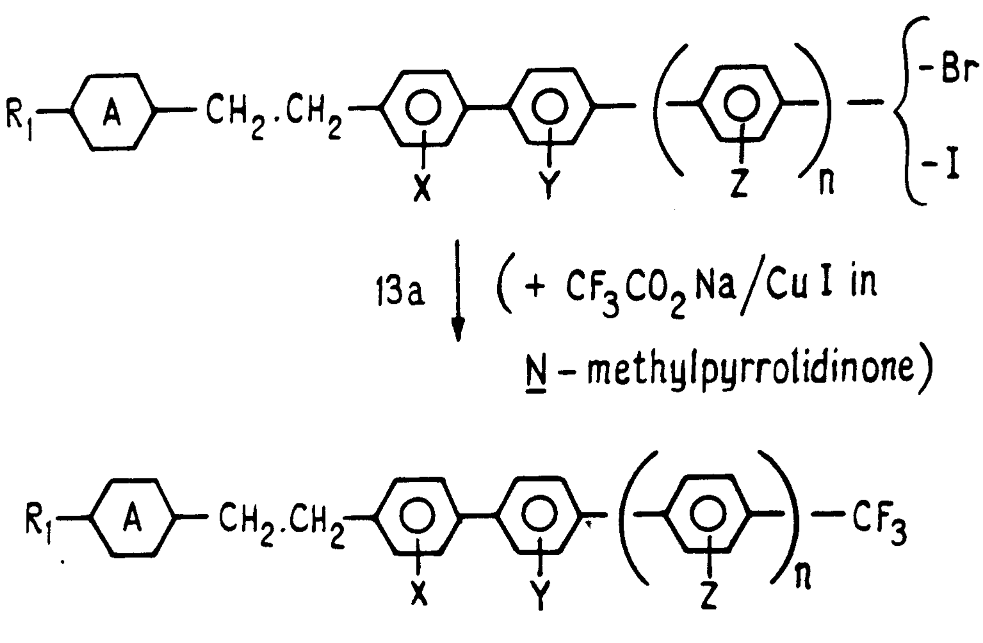


FIG. 13

## Route 14

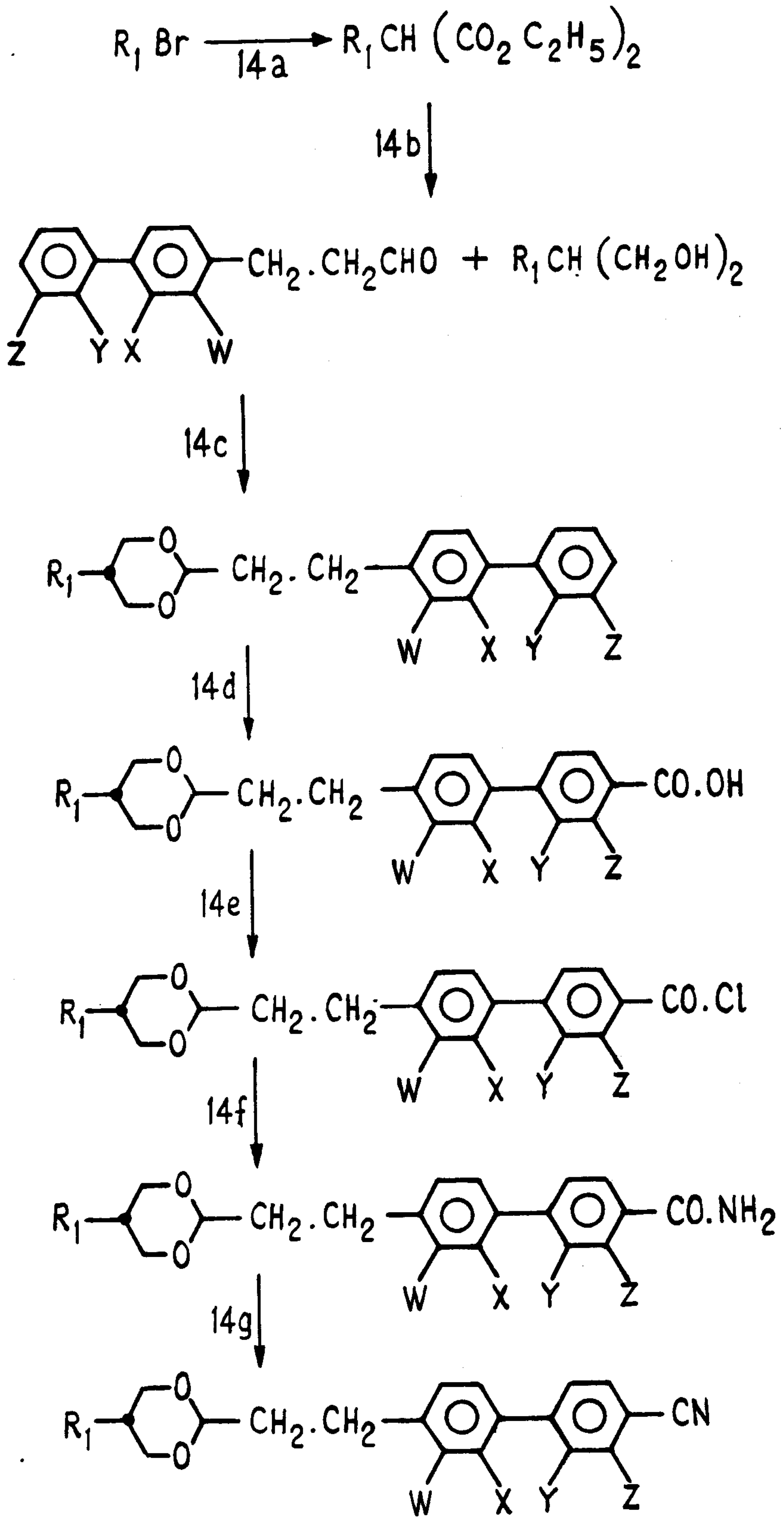


FIG.14

Route 15

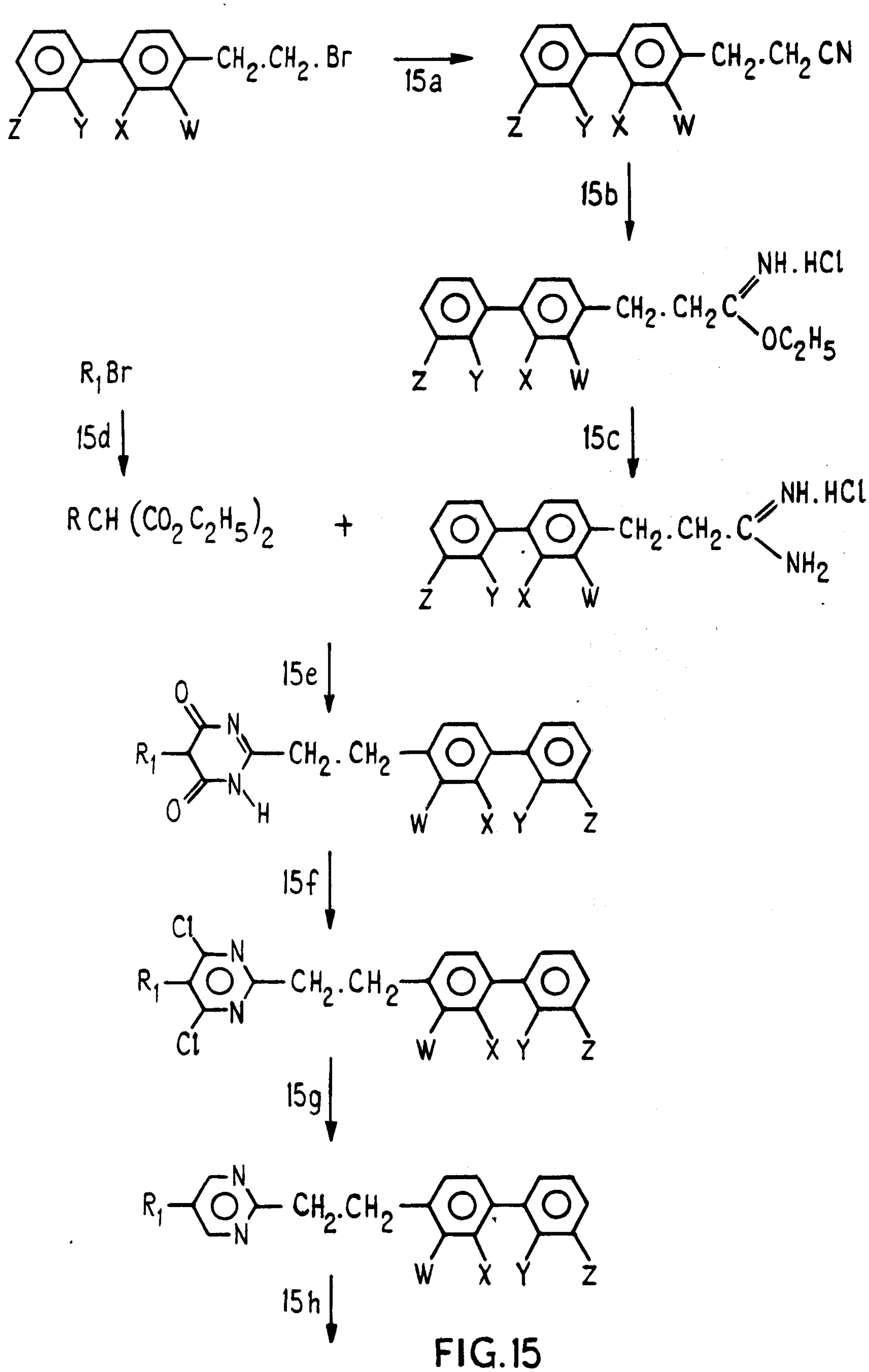


FIG. 15

Route 15 - cont'd

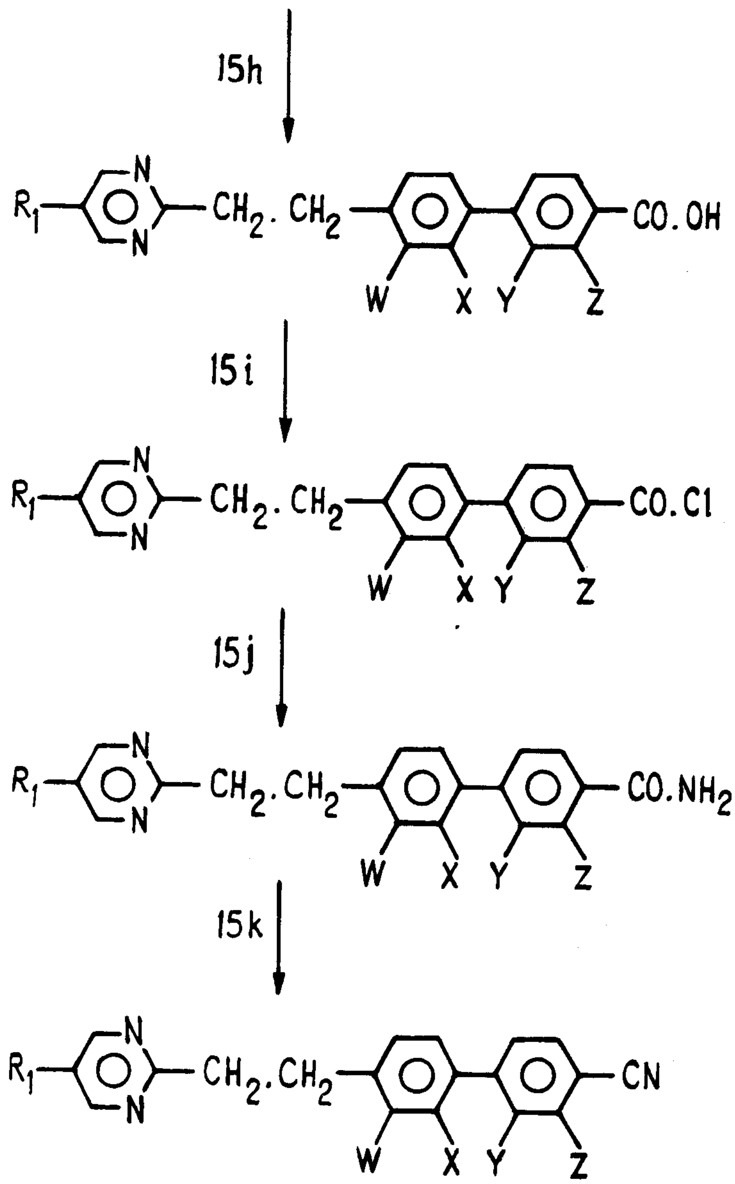


FIG. 15 - cont'd

Route 16

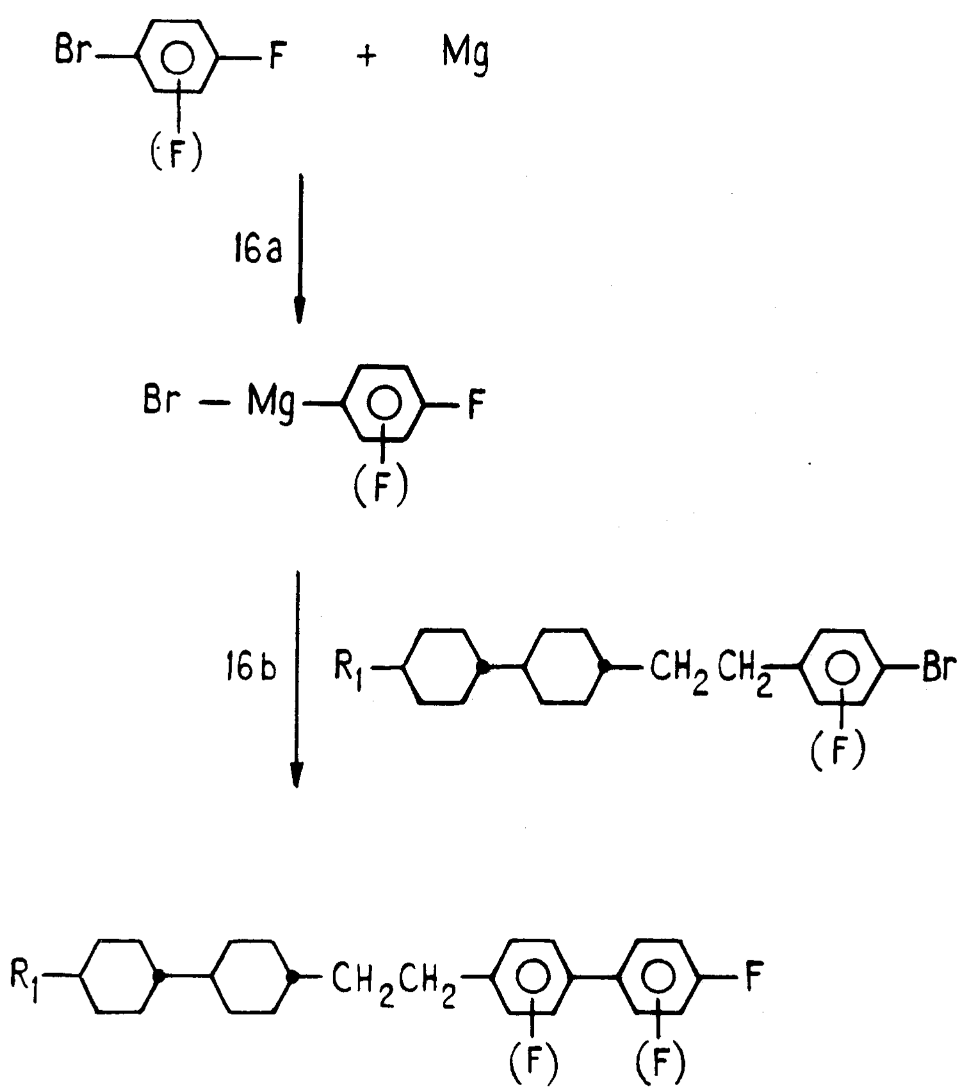


FIG. 16